Europäisches Patentamt

European Patent Office

Office européen des brevets

(19)

(11) Publication number: **0 258 492**
A1

(12) # EUROPEAN PATENT APPLICATION

(21) Application number: 86116938.1

(22) Date of filing: 05.12.86

(51) Int. Cl.³: **C 12 N 15/00**
C 07 K 15/06, C 12 N 1/20
C 12 N 1/18, C 12 P 21/00
C 12 Q 1/68, A 61 K 37/02

(30) Priority: 21.08.86 EP 86111581

(43) Date of publication of application:
09.03.88 Bulletin 88/10

(84) Designated Contracting States:
GB

(71) Applicant: Kishimoto, Tadamitsu, Prof.
Division of Cellular Immunology Institute of Molecular
and Cellular Biology University Osaka
1-3, Yamadaoka Suita Osaka 565(JP)

(72) Inventor: Kishimoto, Tadamitsu, Prof. Dr.
3-5-31, Nakaro Tondabayashi
Osaka 584(JP)

(72) Inventor: Suemura, Masaki, Dr.
9-5, Himemuro Ikeda
Osaka 563(JP)

(72) Inventor: Kikutani, Hitoshi, Dr.
2-1-26, Esaka Suita
Osaka 564(JP)

(72) Inventor: Barsumian, Edward L., Dr.
3-1-503, Senba-Nishi Mino
Osaka 562(JP)

(74) Representative: Laudien, Dieter, Dr. et al,
Boehringer Ingelheim Zentrale GmbH ZA Patente
Postfach 200
D-6507 Ingelheim am Rhein(DE)

(54) Human low affinity Fc epsilon-receptor, the isolation, the recombinant preparation and purification thereof.

(57) The present invention is concerned with human low affinity Fc -receptor and the water-soluble part thereof starting with amino acids from about 50 to about 150 of the human low affinity Fc -receptor, preferably with the N-terminal Met-Glu-Leu-Gln-Val-Ser-Ser-Gly-Phe-Val-, their isolation, recombinant preparation and purification.

The prepared human low affinity Fc -receptor, preferably the water-soluble part thereof, is suitable for the treatment of local and allergic reactions induced by expression of IgE and may be incorporated into the suitable pharmaceutical compositions.

EP 0 258 492 A1

./...

Fig.15

12/036IP/2-Mi/Fl/sch

# Human low affinity $Fc_\varepsilon$-receptor, the isolation, the recombinant preparation and purification thereof

Receptors for the Fc-portion of immunoglobuline (FcR) are expressed by various hematopoietic cell lineages and provide an important link between antibody molecules and their effector functions, such as internalization of the ligand-receptor complexes, antibody-mediated cytolysis of target cells and the release of inflammatory mediators. The expression of Fc-receptors has also been demonstrated on T and B lymphocytes, suggesting a possible role for FcR in the regulation of the immune response as well as the involvement of immunoglobulin (Ig)-binding factors, such as IgE-, IgA- and IgG-binding factors, in isotype specific regulation of the antibody response. At present, no Fc-receptors or the genes encoding Fc-receptors have yet been isolated, although two kinds of Fc-receptors for IgE ($Fc_\varepsilon R$) are known which differ in structure and function, namely

a) high affinity $Fc_\varepsilon R$ on basophils and mast cells and
b) low affinity $Fc_\varepsilon$-receptors on lymphocytes and monocytes.

$Fc_\varepsilon$-receptor was found to be an insoluble membrane protein with the unusual and unexpected characteristic of having a N-terminal in the cytoplasm and a C-terminal outside of the cell, contrary to known receptors. Moreover, an increase of water-soluble $Fc_\varepsilon R$ as a complex with IgE was observed in the serum of atopic patients.

This invention is concerned with human low affinity $Fc_\varepsilon$-receptor and the water-soluble part thereof starting with amino acids from about 50 to about 150 of the whole $Fc_\varepsilon$-receptor, preferably with the N-terminal Met-Glu-Leu-Gln-Val-Ser-Ser-Gly-Phe-Val-, their isolation and purification, whereby the following aspects are described in detail below:

a) Isolation and purification of the water-soluble part of IgE binding factor ($Fc_\varepsilon$R) secreted or shed by lymphoblastoid cells;

b) Partial sequencing of the water-soluble part of the human low affinity $Fc_\varepsilon$-receptor isolated according to a) by means of hydrolysis, isolating the thus obtained fragments and determination of the sequences of the thus obtained fragments;

c) Preparation of two hybridization probes:

Probe 1: Preparation of $Fc_\varepsilon^+L$ cell transformant specific cDNA by using multiple cyclic substraction with Ltk⁻cell mRNA;

Probe 2: Preparation of an oligonucleotide encoding one of the said partial sequences isolated according to b), preferably of a mixture of oligonucleotides of formula

$$3'-TT^T_C \ ACC \ TA^T_A G \ TT^A_G \ AA^A_G \ GT \ -5'$$

encoding for the amino acid sequence of formula

Lys-Trp-Ile-Asn-Phe-Gln;

d) Isolating and identifying expression vehicles containing the gene coding for human low affinity $Fc_\varepsilon$-receptor, comprising the steps

synthesizing cDNA from a RNA matrix derived from lymphoblastoid cells producing $Fc_\varepsilon$-receptor mRNA,

incorporating said synthesized cDNA in expression vehicles to form an expression vehicle bank,

hybridizing said incorporated cDNA to identify those expression vehicles which contain a gene coding for $Fc_\varepsilon$-receptor, with two labelled probes comprising cDNA specific to $Fc_\varepsilon R^+L$ cell and an oligonucleotide common to the gene of low affinity $Fc_\varepsilon$-receptor, and

replication of the thus obtained $Fc_\varepsilon$-receptor gene;

e) Expression of the $Fc_\varepsilon R$ cDNA;

f) Determination of the gene coding for the human low affinity $Fc_\varepsilon$-receptor utilizing isolated cDNA sequence obtained from the vehicles from operation e) according to the sequencing methods of Sanger et al. and the chemical cleavage method of Maxam and Gilbert;

g) Expression of $Fc_\varepsilon R$ mRNA;

h) Preparation of an expression vector containing the DNA sequences coding for a water-soluble fragment of the $Fc_\varepsilon$-receptor and expressing said soluble fragment in microorganisms or in mammalian cells; and

i) Use of the expressed polypeptides for the treatment of local and systemic IgE-allergic reactions and the pharmaceutical compositions containing these polypeptides.

a) <u>Isolation and purification of water-soluble part of $Fc_\varepsilon R$</u>

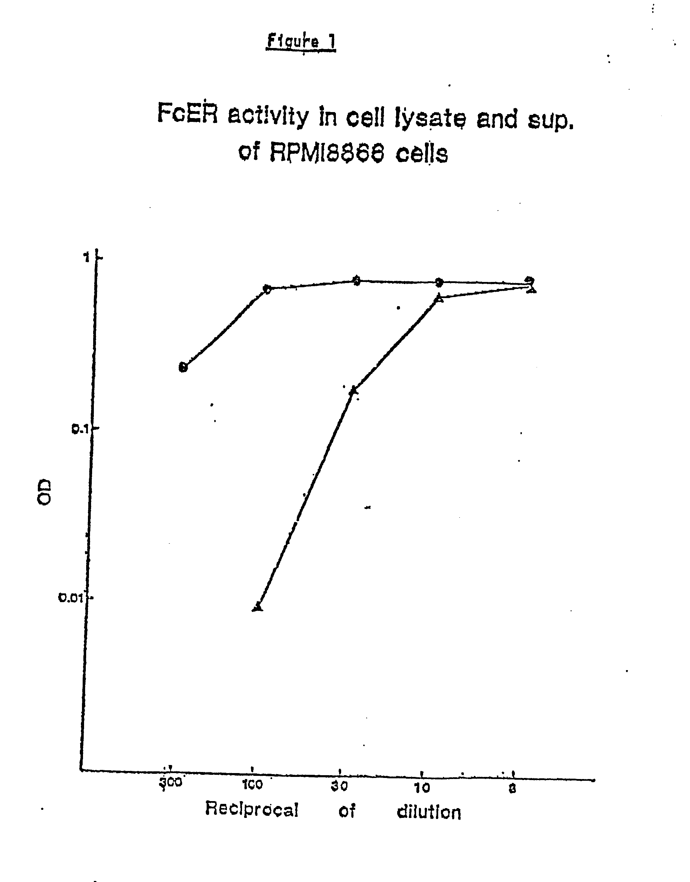

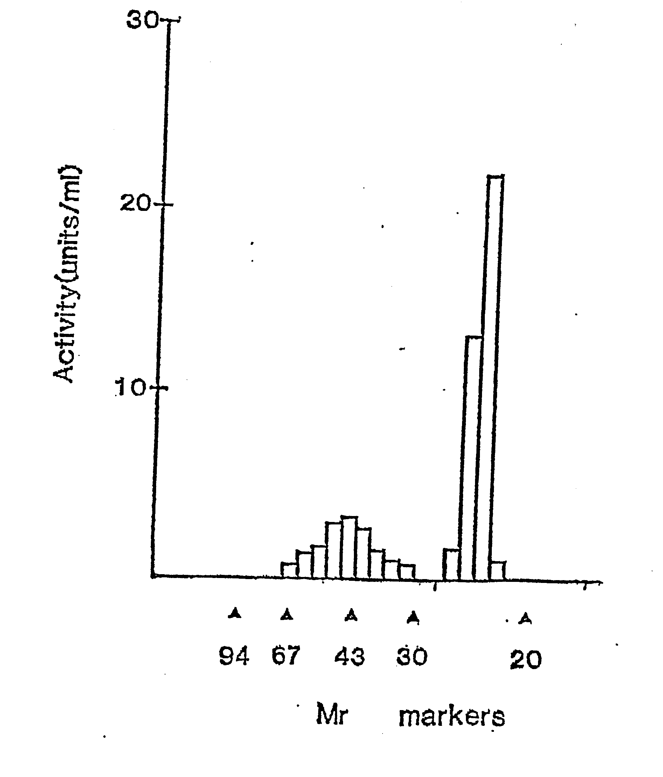

Human B lymphoblastoid cells such as RPMI 8866 cells secrete $Fc_\varepsilon R$ of about 46 kd on their surface and release a species of about 25 kd into the culture supernatent. It has been found, that the $Fc_\varepsilon R$ activity secreted or shed by lymphoblastoid cells, e.g. RPMI-8866 cells, as detected by the ELISA method (Figure 1) utilizing two different monoclonal antibodies (see European Patent Application No. 86 110 420.6 of the same applicant, filed on July 29, 1986) was higher in the concentrated culture supernatant compared to NP-40 detergent solubilized membrane receptors even though an equal number, e.g., $10^5$ cells/ml were utilized to prepare $Fc_\varepsilon R$. Furthermore, when affinity purified supernatants were chromatographed on SDS-PAGE under non-reducing conditions and $Fc_\varepsilon R$ activity eluted from portions of the gel corresponding to defined molecular weight, activity was observed (Figure 2) in the 45-46 kd and 24-25 kd regions. In fact the concentrated culture supernatants contained a higher proportion of activity in the 25 kd region. Therefore serum-free culture supernatants were used as the source of the receptor.

For the sequential immunoaffinity purification of $Fc_\varepsilon R$ with a molecular weight of about 25 kd immunoaffinity columns were used which were prepared utilizing 10 mg/ml of purified monoclonal antibody coupled to Sepharose 4B beads (Pharmacia, Piscataway, N.J.) as described by the manufacturer.

The sequential adsorption of 200-250 x concentrated culture supernatant on BSA-Sepharose, transferrin Sepharose and normal mouse IgG-Sepharose yielded about 70 percent of the original activity without however, improving on the specific activity. As shown in Table 1 following preadsorption the receptor material was allowed to bind to 3-5-Sepharose column for 4-16 hours, the total activity of the acetic acid eluate was reduced but the specific activity was increased to 83 units/µg and the purification was 190 fold. Further purification of the receptor by HPLC reverse-phase chromatography on C-4 column using a linear gradient of 0-65 % acetonitril and 0,1 % trifluoracetic acid increased the specific activity to 1630 units/µg and the receptor was purified 3710 fold. However; the final recovery was only 33 percent of the original. As seen in Table 1, a similar purification scheme was used for detergent-solubilized membrane $Fc_\varepsilon R$ but the specific activity obtained was consistently lower than that observed with culture supernatants. It is important to note that the choice of elution buffer was critical to the level of recovery, 2.5 percent acetic acid elution with rapid neutralization was important in the final yield of the receptor.

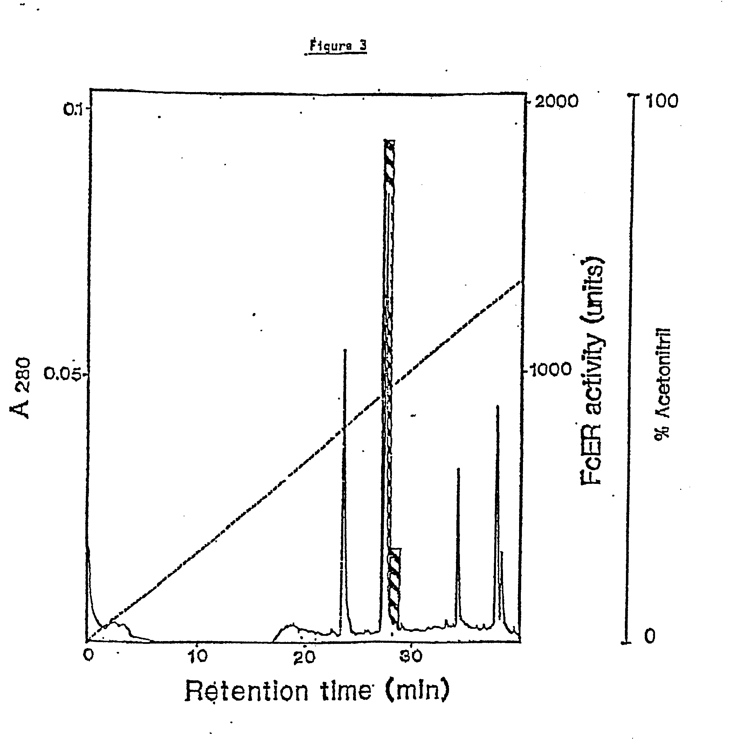

As indicated in Table 1 immunoaffinity purification of $Fc_\varepsilon R$ utilizing the specific monoclonal antibodies in the solid phase was not sufficient to obtain pure receptor as measured by a single band on SDS-PAGE. Therefore, freshly eluted $Fc_\varepsilon R$ was further purified by means of HPLC Reverse Phase Purification. The freshly eluted $Fc_\varepsilon R$ was loaded preparatively on a C-4 column and chromatographed utilizing a linear gradient of 0-65 percent acetonitril the chromatographic profile is shown on Figure 3, fractions obtained at various retention times were tested by ELISA for $Fc_\varepsilon R$ activity. It was found that the bulk of the $Fc_\varepsilon R$ was eluted by acetonitril at between 44 and 45 percent concentration. When 0.5 ml samples were collected the $Fc_\varepsilon R$ activity corresponded to the hatched peak indicated in Figure 3. The rechromatographic analysis of the active fraction showed a single sharp peak indicating the presence of a homogenous mixture of receptor. The fractions obtained at different retention times were also monitored by SDS-PAGE analysis.

Therefore, the concentrated culture supernatant (crude sup) containing the putative $Fc_\varepsilon R$ and the material eluted from immunoaffinity and C-4 HPLC column were tested after dialysis and lyophilization on a 10 percent SDS-PAGE as described below. The results indicate the presence of multiple bands in the lane of the crude concentrated sup. A broad band corresponding to 22-24 kd can be seen.

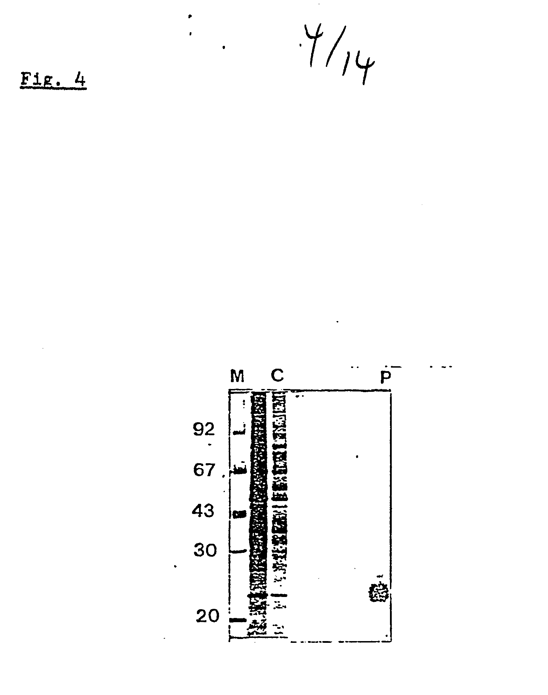

The receptor moiety collected after sequential purification on non-specific and specific immunoaffinity gels still shows multiple bands even though the activity of such eluates is substantially higher than that of crude material. The $Fc_\epsilon R$ activity (Figure 4) obtained after C-4 HPLC purification showed a single band corresponding to 25 kd and the material purified in this fashion showed very high activity in the ELISA assay utilizing the monoclonal antibodies. It is important to note that the band of 25 kd corresponds to the minor species of $Fc_\epsilon R$ detected by the same monoclonal antibodies after surface iodination and immunoprecipitation of the $Fc_\epsilon R$ utilizing the same antibodies, suggesting that the 46 and 25 kd moieties are antigenically identical, and the latter being the water-soluble part of $Fc_\epsilon R$.

As the purification of the IgE binding activity from RPMI-8866 cell culture supernatants entailed many steps, it was important to check for the immunological activity of the putative $Fc_\epsilon R$ at various stages of purification. Equal amounts of HPLC purified $Fc_\epsilon R$ were reapplied to specific 3-5-immunoaffinity and non-specific NMIg-Sepharose gels and the activity was monitored in the effluent and eluate of these gels. The results shown in Table 2 indicate that the purified material obviously binds to the specific column and almost all the activity is recovered in the eluate, compared to the non-specific gel where all the disposable activity is found in the effluent and a minor portion in the eluate. It can also be seen that a good portion of the activity is loosely associated to the non-specific gel and lost during washing.

b) Partial sequencing of water-soluble part of $Fc_\epsilon R$

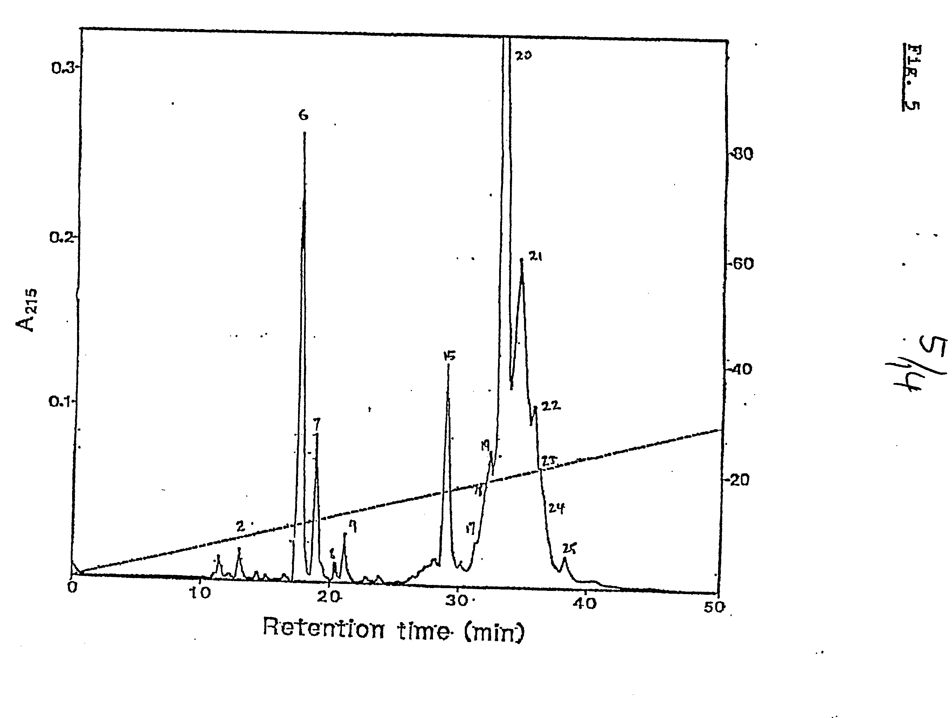

$Fc_\epsilon R$ prepared after sequential purification of concentrated cell culture supernatant utilizing immunoaffinity gels and C-4 HPLC corresponding to a single band on SDS-PAGE and active in the ELISA assay was subjected to amino acid sequencing. Two different proteolytic preparations were made utilizing trypsin and lysylendopeptidase. A total of 11 and 12 fragments were obtained with trypsin and lysylendopeptidase treatment respectively. The HPLC profile of the lysylendopeptidase fragmentation shown on figure 5 indicate 12 major peaks corresponding to defined fragments of $Fc_\varepsilon R$. The following selected fragments were obtained:

Met-Glu-Leu-Gln-Val-Ser-Ser-Gly-Phe-Val-(N-terminal),

Gly-Glu-Phe-Ile-Trp-Val-Asp-Gly-Ser-His-Val-Asp-Tyr-Ser-Asn-Trp-Ala-Pro-Gly-Glu-Pro-Thr-,

Lys-His-Ala-Ser-His-Thr-Gly-Ser-Trp-Ile-Gly-Leu-Arg-Asn-Leu-Asp-Leu-Lys- and

Lys-Trp-Ile-Asn-Phe-Gln-.

## c) Preparation of the hybridization probes

Probe I (cDNA specific to $Fc_\varepsilon R$ positive L cells):

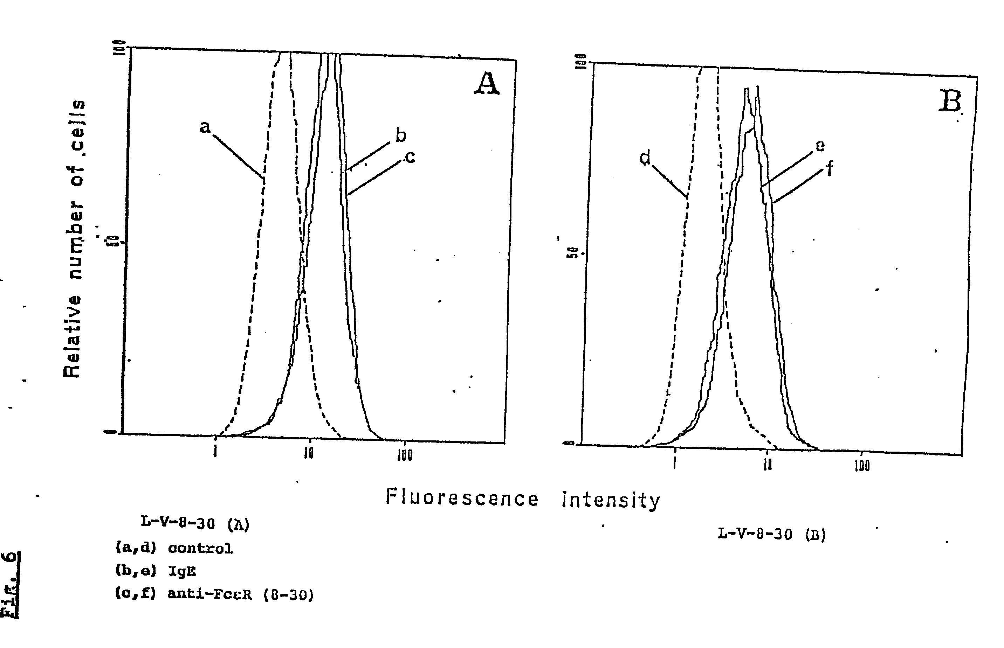

Thymidine kinase (TK) deficient L cells, Ltk⁻ cells, were co-transfected with high molecular weight DNA from RPMI-8866 cells and the Herpes-simplex virus-derived TK gene. After HAT selection, TK positive transformants were stained with biotinated anti-$Fc_\varepsilon R$ antibody (8-30) and FITC avidin and sorted by a cell sorter. $Fc_\varepsilon R$ positive L cells were enriched by several cycles of sorting. Two L cell transformed lines, L-V-8-30 and L-VI-8-30, which express $Fc_\varepsilon R$ detected by anti-$Fc_\varepsilon R$(8-30), were established from two independent transfection experiments. Fig. 6 shows FACS analysis of L-V-8-30 cells which were stained with anti-$Fc_\varepsilon R$ as well as human IgE.

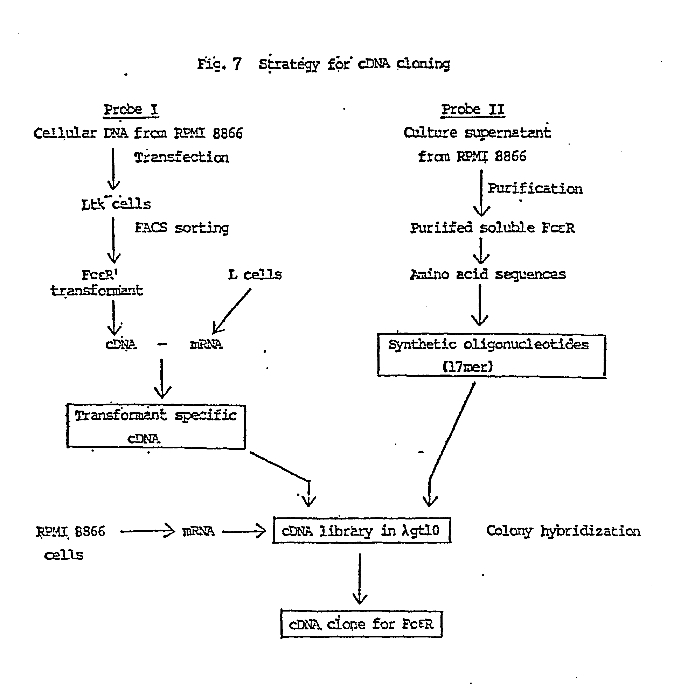

The total RNA was prepared from L-V-8-30 cells by guanidine isothiocyanate/cesium chloride method (see Biochemistry 18, 5294 (1979)). The DNA complementary to mRNA from L-V-8-30 cells was synthesized using the enzyme reverse transcriptase on an oligo (dt) primer. The cDNA was labeled by incorporation of $\alpha$-$^{32}$P-deoxy CTP in this reaction. The $^{32}$P labelled cDNA was mixed and hybridized with 10 fold excess poly(A)$^{+}$ RNA derived from Ltk$^{-}$ cells. The above mixture was applied on hydroxyapatite column and the unbound single strand cDNA was rehybridized with poly(A)$^{+}$ RNA from Ltk$^{-}$ cells. The single strand cDNA which is specific to Fc$_{\xi}$R positive L cell transformant was used as probe to detect the gene for Fc$_{\xi}$R in $\lambda$gt 10 library (Fig. 7).

Probe II: A mixture of oligonucleotides of formula

$$3'-TT^{T}_{C}\ ACC\ TA^{T}_{A}G\ TT^{A}_{G}\ AA^{A}_{G}\ GT\ -5'$$

was synthesized by means of an ADI-synthesizer at the 0,2 µMol level according to known methods. The obtained oligonucleotide encoding partially for the amino acid sequence of formula

Lys-Trp-Ile-Asn-Phe-Gln

was used as labeled probe to detect the gene for Fc$_{\xi}$-receptor in an expression vehicle.

d) Isolation and identification of expression vehicles containing the gene coding for human low affinity Fc$_{\xi}$R

The exponentially growing RPMI-8866 cells are disrupted in guanidium isothiocyanate solution. The mRNA is isolated by centrifugation on cesium chloride gradient, phenol extraction and ethanol precipitation. Then, poly(A)$^{+}$ RNA is isolated by oligo (dt) cellulose chromatography.

The construction of double-stranded cDNA is carried out according to Gubler and Hoffman (Gene $\underline{25}$, 263 (1983)). The poly(A)$^+$ RNA is used as a matrix for the preparation of single-strand cDNA by using reverse transcriptase and an oligo (dt) primer. After treatment with RNase H, the second strand of DNA is synthesized by means of DNA polymerase I. The synthesis of first and second strand of DNA was carried out in a solution containing a mixture of deoxynucleotide triphosphate of adenosine, thymidine, guanosine and cytidine. The double stranded cDNA mixture obtained was then modified by means of the enzyme T4 DNA polymerase to remove any small remaining 3' overhangs from the first strand cDNA. The EcoRI linkers were added and ligated to the double-stranded cDNA by using the enzyme T4 ligase. The cDNA longer than 1000 bp are fractionated and excess linkers were removed by a Bio-Gel A-50 m column chromatography. The size fractionated cDNA were ligated to EcoRI digested $\lambda$gt 10 phage vector DNA. The $\lambda$gt 10 vectors containing the cDNA were packaged in vitro and infected to Escherichia coli strain 0600 hfl.

Among the plaques thus obtained, those which contain sequences specific to Fc$_\epsilon$R were identified by colony hybridization, whereby two different probes were used:

1. Fc$_\epsilon$R$^+$ transformant-specific cDNA.

2. Radioactively labeled synthetic oligonucleotides of formula

$$3'\text{-}TT^{T}_{C}\ ACC\ TA^{T}_{A}G\ TT^{A}_{G}\ AA^{A}_{G}\ GT\ -5'$$

23 from approximately 300 000 clones hybridizing with both probes were identified and all cDNA inserts hybridized to each other. Among the cDNA's in those clones, the largest cDNA insert (approximately 1600 kb) was elected.

The EcoRI insert from the λgt 10 recombinant DNA clone was labeled by nick translation using $\alpha$-$^{32}$P-dCTP and analysed by Northern hybridization with mRNA from various cells including RPMI-8866, Daudi, CEM, Fc$_\varepsilon$R$^+$ L cells and Ltk$^-$ cell. The insert hybridized only with mRNA from Fc R positive cells such as RPMI-8866 and Fc$_\varepsilon$R$^+$ L cells. This insert was cloned in an EcoRI site of pGEM4 vector (Promega Biotec), named as pFc$_\varepsilon$R-1 and propagated.

e) Expression of the Fc$_\varepsilon$R cDNA:

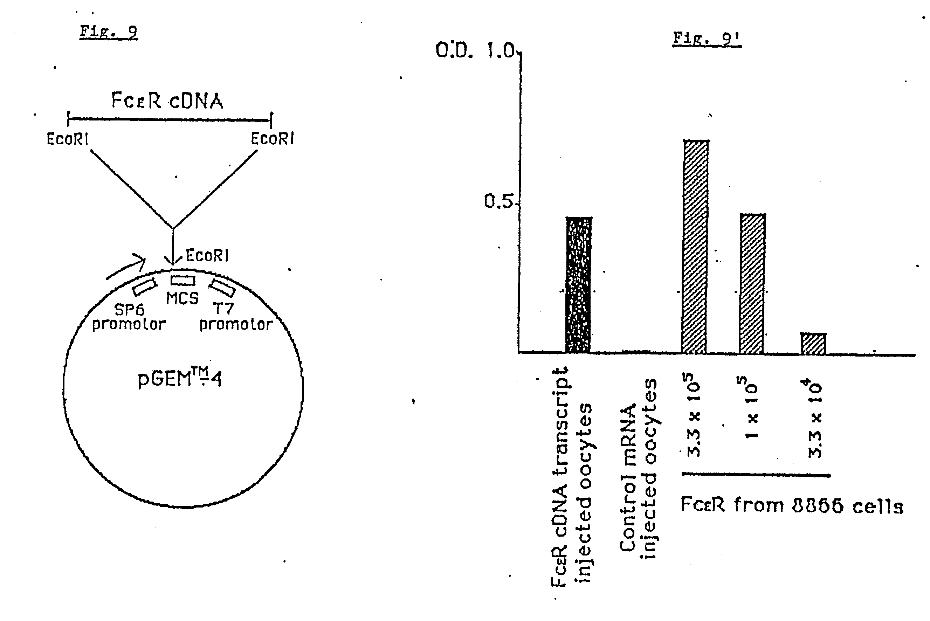

The EcoRI insert containing Fc$_\varepsilon$R cDNA, which was isolated from the above described λgt10 clone, was ligated to EcoRI digested pGEM$^{TM}$4 plasmid vector (see Fig. 9), named as LE392 and deposited in E.coli on August 01, 1986 under number FERM BP-1116 (Fermentation Research Institute, Agency of Industrial Science and Technology, Japan) according to the convention of Budapest. Since this vector contains both SP6 and T7 promotors in opposite orientation to each other, Fc$_\varepsilon$R cDNA can be readily transcribed into mRNA either by SP6 or T7 RNA-polmerase. Therefore, pGEM4-DNA containing Fc$_\varepsilon$R cDNA was digested with BamHI and the obtained plasmid DNA was used as a template to synthesize the mRNA by SP6 RNA polymerase, and the resulting RNA (5 µg) was injected into Xenopus oocytes. After 2 days of incubation, the oocytes were lysed and the presence of Fc$_\varepsilon$R was determined by an enzyme linked immunosorbent assay (ELISA) utilizing two anti-Fc$_\varepsilon$R antibodies, 3-5 and 8-30, which recognize different epitopes on Fc$_\varepsilon$R. As shown in Fig. 9, the lysate of ten oocytes injected with the RNA transcript of pFc$_\varepsilon$R-1 showed Fc$_\varepsilon$R levels comparable to that derived from $1\times10^5$ RPMI-8866 cells. On the other hand, the lysate of mock-injected oocytes did not show any activity. This result indicates that the product of pFc$_\varepsilon$R-1 cDNA shares the two different antigenic determinants with Fc$_\varepsilon$R recognized by the monoclonal antibodies.

0258492

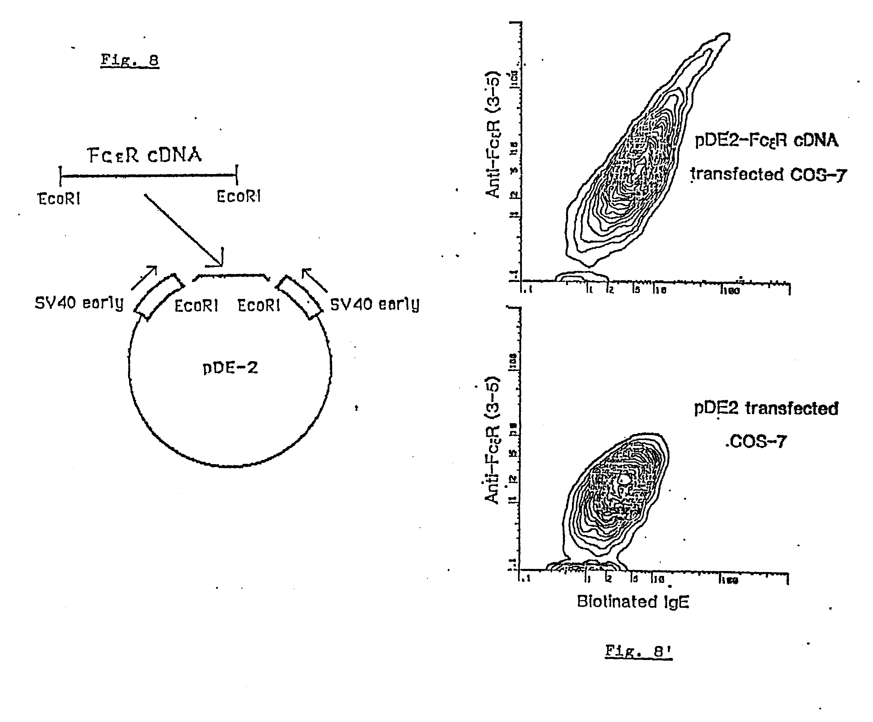

A further expression vector, for example pDE2 (see Japanese Patent Publication 1986/88879 from May 7, 1986), which contains two SV40 early promotors in opposite orientation to each other to ensure the cDNA expression in either orientation (Fig. 8) was employed to confirm that pFc$_\varepsilon$R-1 includes the entire coding sequence of Fc$_\varepsilon$R. The segment of DNA between the two SV40 early promoters was removed by EcoRI digestion and replaced with the insert cDNA of pFc$_\varepsilon$R-1 (pDE2-Fc$_\varepsilon$R-1). Cos7 cells were transfected with 2 µg/ml of pDE2 containing Fc$_\varepsilon$R cDNA by the DEAE-dextran method. After 2 days culture, cells were doubly stained with anti-Fc$_\varepsilon$R and human IgE and analysed on a dual laser FACS. As shown in Fig. 8', approximately 30 % of the cells transfected with pDE2-Fc$_\varepsilon$R-1 were labeled by both anti-Fc$_\varepsilon$R and human IgE. Furthermore, the staining with anti-Fc$_\varepsilon$R and human IgE was well correlated, demonstrating that both anti-Fc$_\varepsilon$R and IgE bound to the same molecule(s) that is newly expressed on the surface of transfected cells. Indeed, cells transfected with the control pDE2 vector containing human IFN-ß cDNA did not stain with either anti-Fc$_\varepsilon$R or human IgE. These results confirmed that the isolated cDNA actually encodes the Fc$_\varepsilon$R molecule.

f) Determination of the complete nucleotide sequence of the Fc$_\varepsilon$R cDNA and the deduced protein sequence

The complete nucleotide sequence of the EcoRI insert from pFc$_\varepsilon$R-1 was determined using the dideoxy termination method (see Sanger et al. in Proc. Natl. Acad. Sci. USA 74, 5463-5467 (1977)) and the chemical cleavage method (see Maxam and Gilbert in Proc. Natl. Acad. Sci. USA 74, 560-564 (1977)). The complete nucleotide sequence and the deduced amino acid sequence are shown in Table 3, whereby the coding sequence shows the following formula:

```
                      5                       10                      15
Met Glu Glu Gly Gln Tyr Ser Glu Ile Glu Glu Leu Pro Arg Arg
ATG GAG GAA GGT CAA TAT TCA GAG ATC GAG GAG CTT CCC AGG AGG
TAC CTC CTT CCA GTT ATA AGT CTC TAG CTC CTC GAA GGG TCC TCC


                     20                      25                      30
Arg Cys Cys Arg Arg Gly Thr Gln Ile Val Leu Leu Gly Leu Val
CGG TGT TGC AGG CGT GGG ACT CAG ATC GTG CTG CTG GGG CTG GTG
GCC ACA ACG TCC GCA CCC TGA GTC TAG CAC GAC GAC CCC GAC CAC


                     35                      40                      45
Thr Ala Ala Leu Trp Ala Gly Leu Leu Thr Leu Leu Leu Leu Trp
ACC GCC GCT CTG TGG GCT GGG CTG CTG ACT CTG CTT CTC CTG TGG
TGG CGG CGA GAC ACC CGA CCC GAC GAC TGA GAC GAA GAG GAC ACC


                     50                      55                      60
His Trp Asp Thr Thr Gln Ser Leu Lys Gln Leu Glu Glu Arg Ala
CAC TGG GAC ACC ACA CAG AGT CTA AAA CAG CTG GAA GAG AGG GCT
GTG ACC CTG TGG TGT GTC TCA GAT TTT GTC GAC CTT CTC TCC CGA


                     65                      70                      75
Ala Arg Asn Val Ser Gln Val Ser Lys Asn Leu Glu Ser His His
GCC CGG AAC GTC TCT CAA GTT TCC AAG AAC TTG GAA AGC CAC CAC
CGG GCC TTG CAG AGA GTT CAA AGG TTC TTG AAC CTT TCG GTG GTG


                     80                      85                      90
Gly Asp Gln Met Ala Gln Lys Ser Gln Ser Thr Gln Ile Ser Gln
GGT GAC CAG ATG GCG CAG AAA TCC CAG TCC ACG CAG ATT TCA CAG
CCA CTG GTC TAC CGC GTC TTT AGG GTC AGG TGC GTC TAA AGT GTC


                     95                      100                     105
Glu Leu Glu Glu Leu Arg Ala Glu Gln Gln Arg Leu Lys Ser Gln
GAA CTG GAG GAA CTT CGA GCT GAA CAG CAG AGA TTG AAA TCT CAG
CTT GAC CTC CTT GAA GCT CGA CTT GTC GTC TCT AAC TTT AGA GTC


                    110                     115                     120
Asp Leu Glu Leu Ser Trp Asn Leu Asn Gly Leu Gln Ala Asp Leu
GAC TTG GAG CTG TCC TGG AAC CTG AAC GGG CTT CAA GCA GAT CTG
CTG AAC CTC GAC AGG ACC TTG GAC TTG CCC GAA GTT CGT CTA GAC


                    125                     130                     135
Ser Ser Phe Lys Ser Gln Glu Leu Asn Glu Arg Asn Glu Ala Ser
AGC AGC TTC AAG TCC CAG GAA TTG AAC GAG AGG AAC GAA GCT TCA
TCG TCG AAG TTC AGG GTC CTT AAC TTG CTC TCC TTG CTT CGA AGT


                    140                     145                     150
Asp Leu Leu Glu Arg Leu Arg Glu Glu Val Thr Lys Leu Arg Met
GAT TTG CTG GAA AGA CTC CGG GAG GAG GTG ACA AAG CTA AGG ATG
CTA AAC GAC CTT TCT GAG GCC CTC CTC CAC TGT TTC GAT TCC TAC


                    155                     160                     165
Glu Leu Gln Val Ser Ser Gly Phe Val Cys Asn Thr Cys Pro Glu
GAG TTG CAG GTG TCC AGC GGC TTT GTG TGC AAC ACG TGC CCT GAA
CTC AAC GTC CAC AGG TCG CCG AAA CAC ACG TTG TGC ACG GGA CTT
```

```
                  170                 175                 180
Lys Trp Ile Asn Phe Gln Arg Lys Cys Tyr Tyr Phe Gly Lys Gly
AAG TGG ATC AAT TTC CAA CGG AAG TGC TAC TAC TTC GGC AAG GGC
TTC ACC TAG TTA AAG GTT GCC TTC ACG ATG ATG AAG CCG TTC CCG

                  185                 190                 195
Thr Lys Gln Trp Val His Ala Arg Tyr Ala Cys Asp Asp Met Glu
ACC AAG CAG TGG GTC CAC GCC CGG TAT GCC TGT GAC GAC ATG GAA
TGG TTC GTC ACC CAG GTG CGG GCC ATA CGG ACA CTG CTG TAC CTT

                  200                 205                 210
Gly Gln Leu Val Ser Ile His Ser Pro Glu Glu Gln Asp Phe Leu
GGG CAG CTG GTC AGC ATC CAC AGC CCG GAG GAG CAG GAC TTC CTG
CCC GTC GAC CAG TCG TAG GTG TCG GGC CTC CTC GTC CTG AAG GAC

                  215                 220                 225
Thr Lys His Ala Ser His Thr Gly Ser Trp Ile Gly Leu Arg Asn
ACC AAG CAT GCC AGC CAC ACC GGC TCC TGG ATT GGC CTT CGG AAC
TGG TTC GTA CGG TCG GTG TGG CCG AGG ACC TAA CCG GAA GCC TTG

                  230                 235                 240
Leu Asp Leu Lys Gly Glu Phe Ile Trp Val Asp Gly Ser His Val
TTG GAC CTG AAG GGA GAG TTT ATC TGG GTG GAT GGG AGC CAT GTG
AAC CTG GAC TTC CCT CTC AAA TAG ACC CAC CTA CCC TCG GTA CAC

                  245                 250                 255
Asp Tyr Ser Asn Trp Ala Pro Gly Glu Pro Thr Ser Arg Ser Gln
GAC TAC AGC AAC TGG GCT CCA GGG GAG CCC ACC AGC CGG AGC CAG
CTG ATG TCG TTG ACC CGA GGT CCC CTC GGG TGG TCG GCC TCG GTC

                  260                 265                 270
Gly Glu Asp Cys Val Met Met Arg Gly Ser Gly Arg Trp Asn Asp
GGC GAG GAC TGC GTG ATG ATG CGG GGC TCC GGT CGC TGG AAC GAC
CCG CTC CTG ACG CAC TAC TAC GCC CCG AGG CCA GCG ACC TTG CTG

                  275                 280                 285
Ala Phe Cys Asp Arg Lys Leu Gly Ala Trp Val Cys Asp Arg Leu
GCC TTC TGC GAC CGT AAG CTG GGC GCC TGG GTG TGC GAC CGG CTG
CGG AAG ACG CTG GCA TTC GAC CCG CGG ACC CAC ACG CTG GCC GAC

                  290                 295                 300
Ala Thr Cys Thr Pro Pro Ala Ser Glu Gly Ser Ala Glu Ser Met
GCC ACA TGC ACG CCG CCA GCC AGC GAA GGT TCC GCG GAG TCC ATG
CGG TGT ACG TGC GGC GGT CGG TCG CTT CCA AGG CGC CTC AGG TAC

                  305                 310                 315
Gly Pro Asp Ser Arg Pro Asp Pro Asp Gly Arg Leu Pro Thr Pro
GGA CCT GAT TCA AGA CCA GAC CCT GAC GGC CGC CTG CCC ACC CCC
CCT GGA CTA AGT TCT GGT CTG GGA CTG CCG GCG GAC GGG TGG GGG

Ser Ala Pro Leu His Ser
TCT GCC CCT CTC CAC TCT TGA
AGA CGG GGA GAG GTG AGA ACT
```

The single large open reading frame begins at position 186 and extends 963 nucleotides, encoding 321 amino acids. The isolated partial amino acid sequences of three peptide fragments and the isolated N-terminal Met-Glu-Leu-Gln-Val-Ser-Ser-Gly-Phe-Val-  of purified $Fc_\varepsilon R$ confirm that the longest frame is the coding sequence of $Fc_\varepsilon R$ protein. The hydrophilic N-terminal sequence consisting of 21 amino acids is followed by a hydrophobic region which consists of 26 uncharged amino acids (22-47). The signal sequence, typically located in the N-terminus of most membrane-bound or secretory proteins, was not found. Hence, the hydrophobic stretch of 26 amino acids is likely to be a membrane-embedded region, since the subsequent residues are mostly hydrophilic and no other part of the sequence appears likely to cross the membrane. The N-terminal hydrophilic stretch is terminated by a cluster of very basic amino acid residues (Arg-Arg-Arg-Cys-Cys-Arg-Arg). This cluster of basic amino acids usually found on the cytoplasmic side of membrane proteins is known to be a stop-transfer sequence which has an important role in integration into the lipid bilayer (see Blobel in Proc. Natl. Acad. Sci. USA 77, 1496-1500 (1980) and Schneider et al. in Nature 311, 675-678 (1984)). There is one putative N-linked carbohydrate addition site at position 63 which should be located in the extracellular region for membrane proteins. All of these results demonstrate that $Fc_\varepsilon R$ is oriented with the N-terminus on the cytoplasmic side and the C-terminus outside the cell.

Relatively large amounts of soluble 25 kd $Fc_\varepsilon R$ were found in the culture supernatant of RPMI-8866 cells. The N-terminal amino acid residue (Met) of the soluble $Fc_\varepsilon R$ was found at position 150, and the preceding residue, arginine is a common target for trypsin-like proteases. The C-terminal region (150-321) contains two clusters of cysteins (160, 163, 174 and 191 and 259, 273, 282 and 288) which probably form disulfide bonds and results in a tightly folded structure, which will be resistant to proteolytic enzymes. The C-termi-

```
Lys Trp Ile Asn Phe Gln Arg Lys Cys Tyr Tyr Phe Gly Lys Gly
AAG TGG ATC AAT TTC CAA CGG AAG TGC TAC TAC TTC GGC AAG GGC
TTC ACC TAG TTA AAG GTT GCC TTC ACG ATG ATG AAG CCG TTC CCG

Thr Lys Gln Trp Val His Ala Arg Tyr Ala Cys Asp Asp Met Glu
ACC AAG CAG TGG GTC CAC GCC CGG TAT GCC TGT GAC GAC ATG GAA
TGG TTC GTC ACC CAG GTG CGG GCC ATA CGG ACA CTG CTG TAC CTT

Gly Gln Leu Val Ser Ile His Ser Pro Glu Glu Gln Asp Phe Leu
GGG CAG CTG GTC AGC ATC CAC AGC CCG GAG GAG CAG GAC TTC CTG
CCC GTC GAC CAG TCG TAG GTG TCG GGC CTC CTC GTC CTG AAG GAC

Thr Lys His Ala Ser His Thr Gly Ser Trp Ile Gly Leu Arg Asn
ACC AAG CAT GCC AGC CAC ACC GGC TCC TGG ATT GGC CTT CGG AAC
TGG TTC GTA CGG TCG GTG TGG CCG AGG ACC TAA CCG GAA GCC TTG

Leu Asp Leu Lys Gly Glu Phe Ile Trp Val Asp Gly Ser His Val
TTG GAC CTG AAG GGA GAG TTT ATC TGG GTG GAT GGG AGC CAT GTG
AAC CTG GAC TTC CCT CTC AAA TAG ACC CAC CTA CCC TCG GTA CAC

Asp Tyr Ser Asn Trp Ala Pro Gly Glu Pro Thr Ser Arg Ser Gln
GAC TAC AGC AAC TGG GCT CCA GGG GAG CCC ACC AGC CGG AGC CAG
CTG ATG TCG TTG ACC CGA GGT CCC CTC GGG TGG TCG GCC TCG GTC

Gly Glu Asp Cys Val Met Met Arg Gly Ser Gly Arg Trp Asn Asp
GGC GAG GAC TGC GTG ATG ATG CGG GGC TCC GGT CGC TGG AAC GAC
CCG CTC CTG ACG CAC TAC TAC GCC CCG AGG CCA GCG ACC TTG CTG

Ala Phe Cys Asp Arg Lys Leu Gly Ala Trp Val Cys Asp Arg Leu
GCC TTC TGC GAC CGT AAG CTG GGC GCC TGG GTG TGC GAC CGG CTG
CGG AAG ACG CTG GCA TTC GAC CCG CGG ACC CAC ACG CTG GCC GAC

Ala Thr Cys Thr Pro Pro Ala Ser Glu Gly Ser Ala Glu Ser Met
GCC ACA TGC ACG CCG CCA GCC AGC GAA GGT TCC GCG GAG TCC ATG
CGG TGT ACG TGC GGC GGT CGG TCG CTT CCA AGG CGC CTC AGG TAC

Gly Pro Asp Ser Arg Pro Asp Pro Asp Gly Arg Leu Pro Thr Pro
GGA CCT GAT TCA AGA CCA GAC CCT GAC GGC CGC CTG CCC ACC CCC
CCT GGA CTA AGT TCT GGT CTG GGA CTG CCG GCG GAC GGG TGG GGG

Ser Ala Pro Leu His Ser
TCT GCC CCT CTC CAC TCT TGA
AGA CGG GGA GAG GTG AGA ACT
```

However, the before mentioned polypeptide is a preferred one of the water-soluble species of human low affinity Fc$_\varepsilon$R. The corresponding genes can be introduced into organisms under conditions which lead to high yields thereof, as mentioned hereinbefore. Useful hosts and vectors are well known to those of skill in the art, and reference is made, for example, to European Patent Publication 0 093 619 published November 9, 1983.

0258492

nal region (150-321) corresponds therefore, to the soluble $Fc_\varepsilon R$ which is a product of proteolytic cleavage of the membrane-bound $Fc_\varepsilon R$.

g) Expression of the $Fc_\varepsilon R$ mRNA:

The poly(A)[+] RNA was prepared from various types of cells and analyzed for expression of $Fc_\varepsilon R$ mRNA by Northern blotting, whereby a major band of 1,700 b was detected in B lymphoblastoid cell lines (RPMI-8866, RPMI-1788), fetal liver-derived Epstein Barr virus transformed pre B cell line (FL #8-2) and two $Fc_\varepsilon R^+$ L cell transformants, but not in $Fc_\varepsilon R^-$ cells including two Burkitt's lymphoma lines (Daudi and Jijoye), a T cell line (CEM) and a L cell transformant which expresses another B Cell antigen (CD20). Furthermore, $Fc_\varepsilon R$ mRNA was not detected in normal T cells, whereas normal B cells expressed a comparable level of $Fc_\varepsilon R$ mRNA as B lymphoblastoid lines.

h) Preparation of an expression vector containing the DNA-sequences coding for a water soluble fragment of $Fc_\varepsilon$-receptor

From the obtained gene for $Fc_\varepsilon$-receptor (see table 3) were removed by means of a suitable endonuclease the codons coding for the water-insoluble part of $Fc_\varepsilon$-receptor, conveniently the codons for the amino acids from about 50 to 150, preferably from about 150. When introducing the obtained shortened $Fc_\varepsilon$-receptor genes into organisms under condition which lead to high yield thereof, there were obtained the desired water-soluble polypeptides without the above mentioned amino acids. Therefore, the water soluble part of $Fc_\varepsilon$-receptor contains at least the following sequence:

```
                                                    Met
                                                    ATG
                                                    TAC

Glu Leu Gln Val Ser Ser Gly Phe Val Cys Asn Thr Cys Pro Glu
GAG TTG CAG GTG TCC AGC GGC TTT GTG TGC AAC ACG TGC CCT GAA
CTC AAC GTC CAC AGG TCG CCG AAA CAC ACG TTG TGC ACG GGA CTT
```

In general, prokaryotes are preferred for expression. For example, E. coli K12 strain 294 (ATCC No. 31446) is particularly useful. Other microbial strains which may be used include E. coli X1776 (ATCC No. 31537). The aforementioned strains, as well as E. coli W3110 (F⁻, lambda⁻, prototrophic, ATCC No. 27325), bacilli such as Bacillus subtilus, and other enterobacteriaceae such as Salmonella typhimurium or Serratia marcenses, and various Pseudomonas species may be used.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, E. coli is typically transformed using pBR322, a plasmid derived from an Escherichia coli species (Bolivar, et al., Gene 2: 95 (1977)). pBR322 contains genes for ampicillin and tetracyline resistance and thus provides easy means for identifying transformed cells. The pBR322 plasmid, or other microbial plasmids must also contain, or be modified to contain, promotors which can be used by the microbial organism for expression. Those promotors most commonly used in recombinant DNA construction include the beta-lactamase (penicillinase) and lactose promotor systems (Chang et al., Nature 275, 615 (1978); Itakura et al., Science 198, 1056 (1977); Goeddel et al., Nature 281, 544 (1979)) and tryptophan (trp) promotor system (Goeddel et al., Nucleic Acids Res. 8, 4057 (1980); EP-A-0 036 776). While these are the most commonly used, other microbial promotors have been discovered and utilized.

For example, the genetic sequence for Fc$_\varepsilon$R can be placed under the control of the leftward promotor of bacteriophage Lambda ($P_L$). This promotor is one of the strongest known promotors which can be controlled. Control is exerted by the lambda repressor, and adjacent restriction sites are known. A temperature sensitive allele of this repressor gene can be placed on the vector that contains the complete $Fc_\epsilon R$ sequence. When the temperature is raised to 42°C, the repressor is inactivated, and the promotor will be expressed at its maximum level. The amount of mRNA produced under these conditions should be sufficient to result in a cell which contains about 10 % of its newly synthesized RNA originated from the $P_L$ promotor. In this scheme, it is possible to establish a bank of clones in which a functional $Fc_\epsilon R$ sequence is placed adjacent to a ribosome binding sequence, and at varying distances from the lambda $P_L$ promotor. These clones can then be screened and the one giving the highest yield selected.

The expression and translation of the $Fc_\epsilon R$ sequence can also be placed under control of other regulons which may be "homologous" to the organism in its untransformed state. For example, lactose dependent E. coli chromosomal DNA comprises a lactose or lac operon which mediates lactose digestion by expressing the enzyme beta-galactosidase. The lac control elements may be obtained from bacteriophage lambda plaC5, which is infective for E. coli. The phage's lac operon can be derived by transduction from the same bacterial species. Regulons suitable for use in the process of the invention can be derived from plasmid DNA native to the organism. The lac promoter-operator system can be induced by IPTG.

Other promoter-operator systems or portions thereof can be employed as well. For example, the arabinose operator, Colicine $E_1$ operator, galactose operator, alkaline phosphatase operator, trp operator, xylose A operator, tac promotor, and the like can be used.

- 19 -

0258492

The genes can be expressed most advantageously in Escherichia coli when using the promotor-operator system of plasmid pER 103 (see E. Rastl-Dworkin et al. in Gene 21, 237-248 and EP-A-0.115.613) deposited at German Collection of Micoorganisms, Grisebachstraße 8, D-3400 Göttingen, on October 27, 1983 under DSM 2773 according to the Budapest Treaty.

A corresponding expression plasmid, for example, to express the water-soluble part of the human low affinity $Fc_\varepsilon$-receptor with the amino acids 150 to 321 (see table 3) can be prepared as follows:

A plasmid containing the above mentioned promotor-operator system, for example the plasmid pRH 100 (see Example B) was digested with SstI. Subsequently, the 3'-overhangs were removed and the linearised plasmid was dephosphorylated. After purification, e.g. by phenol/chloroform extraction, electrophoresis, electroelution and precipitation, the linearised vector is ligated with an insert obtained as follows:

A plasmid containing part of the human low affinity $Fc_\varepsilon$-receptor gene, for example pGEM4-$Fc_\varepsilon$R, which was obtained by digestion of the EcoRI-insert of the plasmid LE392 with HindIII and by reinsertion of the larger EcoRI/HindIII-fragment into pGEM4 (Promega Biotec, Madison, WI53711, USA) was digested with EcoRI and HindIII. Subsequently, the 5' overhanging ends were made blunt by addition of the Klenow fragment of DNA-polymerase I and all four dXTP's, and the 5'-phosphate groups were removed. After purification of the obtained fragment, this was recut with Sau3A and the larger fragment was isolated. Since this procedure removes not only the 5' upstream region but also the nucleotides for the first 18 N-terminal amino acids of the desired water-soluble part, two oligodeoxynucleotides of formulas

EBI-496:

5'  GAACTGCAGGTGAGCTCTGGTTTCGTTTGCAACACTTGCCCGGAAAAATG        3'

EBI-497:

3'  CTTGACGTCCACTCGAGACCAAAGCAAACGTTGTGAACGGGCCTTTTTAC<u>CTAG</u> 5'
                                                          Sau3A

were synthesized to restore corresponding codons of the formula

```
        GluLeuGlnValSerSerGlyPheValCysAsnThrCysProGluLysTrp
5'  GAACTGCAGGTGAGCTCTGGTTTCGTTTGCAACACTTGCCCGGAAAAATG        3'
3'  CTTGACGTCCACTCGAGACCAAAGCAAACGTTGTGAACGGGCCTTTTTAC<u>CTAG</u> 5'
                                                          Sau3A
```

(without the ATG-codon because this codon is contained in the promotor/operator/linker-system of the plasmid pER 103).

Each of the prepared oligodeoxynucleotides were annealed and ligated to the above obtained Fc$_{\varepsilon}$R-water-soluble fragment gene. After heat denaturation of the used T4-DNA ligase, T4-polynucleotidekinase, and ATP was added to phosphorylate the 5' ends of the DNA.

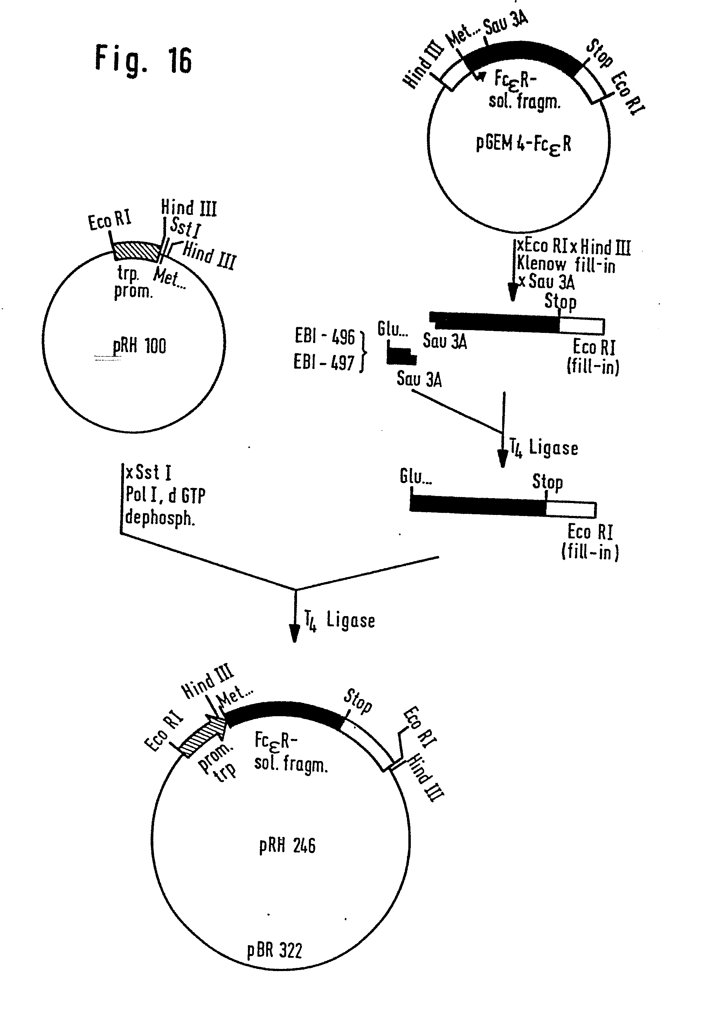

After purification of the insert by means of agarose gel electrophoresis, this insert was ligated with the linearised vector DNA. The obtained ligation solution was transformed in E.coli HB 101 and some of the ampicillin resistent colonies were isolated. These plasmids were checked by means of restriction enzyme analysis for the correctness of their construction. One plasmid was selected and designated as pRH 246 (see Fig. 16).

In addition to prokaryotes, eukaryotic microbes, such as yeast cultures may also be used. Saccharomyces cerevisiae is the most commonly used among eukaryotic microorganisms, although a number of other species are commonly available. For expression in Saccharomyces, the plasmid YRp7, for example (Stinchcomb, et al., Nature 282, 39, (1979); Kingsman et al., Gene 7, 141 (1979); Tschumper, et al., Gene 10, 157 (1980)), plasmid YEp13 (Bwach et al., Gene 8, 121-133 (1979)) and plasmid pJDB207 (see V.D. Beggs et al. in "Gene cloning in yeast", Ed.R. Williamson: Genetic engineering Vol. 2, 175-203 (1981), Academic Press, London; deposited on December 28, 1984 under the DSM 3181 at German Collection of Microorganisms, Grisebachstraße 8, D-3400 Göttingen, according to the Budapest Treaty) are commonly used. The plasmid YRp7 contains the TRP1 gene which provides a selection marker for a mutant strain of yeast lacking the ability to grow in tryptophan, for example ATCC No. 44076. The presence of the TRP1 lesion as a charactristic of the yeast host cell genome then provides an effective environment for detecting transformation by growth in the absence of tryptophan. Similarly, the plasmid YEp13 contains the yeast LEU2 gene which can be used for complementation of a LEU2 minus mutant strain.

Suitable promoting sequence in yeast vectors include the 5'-flanking region of the genes for ADH I (Ammerer, G., Methods of Enzymology 101, 192-201 (1983)), 3-phosphoglycerate kinase (Hitzemann, et al., J. Biol. Chem. 255, 2073 (1980)) or other glycolytic enzymes (Kawasaki and Fraenkel, BBRC 108, 1107-1112 (1982)), such as enolase, glyceraldehyde-3-phosphate dehydrogenase, hexokinase, pyruvate decarboxylase, phosphofructokinase, glucose-6-phosphate isomerase, phosphoglucose isomerase, and glucokinase. In constructing suitable expression plasmids, the termination sequences associated with these genes are also ligated into the expression vector 3' end of the sequence desired to be expressed, to provide polyadenylation of the mRNA and termination.

Other promotors, which have the additional advantage of transcription controlled by growth conditions are the promotor regions of the genes for alcohol dehydrogenase-2, iso-cytochrome C, acid phosphatase, degradative enzymes associated with nitrogen metabolism, the aforementioned glyceraldehyde-3-phosphate dehydrogenase, and enzymes responsible for maltose and galactose utilization. Promotors which are regulated by the yeast mating type locus, such as the promotors of the genes BARI, MR-alpha-1, STE2, STE3, STE5 can be used for temperature regulated system by using temperature dependent siv mutations (Rhine, Ph. D. Thesis, University of Oregon, Eugene, Oregon (1979), Herskowitz and Oshima in The Molecular Biology of the Yeast Sacharomyces, Part I, 181-209 (1981), Cold Spring Harbor Laboratory). These mutations directly influence the expressions of the silent mating type cassettes of yeast, and therefore indirectly the mating type dependent promotors.

Generally, however, any plasmid vector containing a yeast compatible promotor, origin of replication and termination sequences is suitable.

However, the genes, preferably the genes for the water-soluble part of the human low affinity $Fc_\varepsilon$-receptor with the amino acids 150 to 321 (see table 3), can be expressed in yeast most advantageously when using the ADHI-promotor together with the ADHI-terminator. For example, the preparation of a suitable yeast expression vector can be carried out by preparing

a) a plasmid containing the yeast ADHI-terminator,

b) a plasmid containing the yeast ADHI-promotor and the yeast ADHI-terminator (see J. Biol. Chem. 257, 3018-3025 (1982)),

0258492

- 23 -

c) a plasmid containing the yeast ADHI-promotor, a gene
coding for the yeast mating factor α leader peptide (MFα
leader sequence) (see Cell 30, 933-943 (1982)), a multicloning site and the yeast ADHI-terminator,

d) a plasmid containing the ADHI-promotor, the coding sequence for the water-soluble part of human low affinity
$Fc_\xi$-receptor and the ADHI-terminator,

e) a plasmid containing the yeast ADHI-promotor, a yeast
mating factor α leader gene, the gene for the water-soluble
part of human low affinity $Fc_\xi$-receptor, a multicloning site
and the yeast ADHI-terminator, and

f) transforming the obtained plasmid DNA in a suitable yeast
vector, whereby a plasmid containing the expression cassette
without the MFα leader sequence and a plasmid containing the
expression cassette with the MFα leader sequence are obtai-
ned.

Description of the procedures a to f:

a) The ADHI-terminator can be isolated from a plasmid containing this promotor, for example from the plasmid pJD14 (see
J.L. Bennetzen et al. in J. Biol. Chem. 257, 3018-3025
(1982)). The ADHI-terminator was subcloned in plasmid pUC18
(Pharmacia P-L, # 27-4949-01) as HindIII-SphI fragment. During subcloning, the HindIII site was destroyed by a fill-
in-reaction and after addition of SalI linker, the plasmid
pWS214S4 was obtained. After digestion of pWS214S4 with SphI
and SalI, the smaller fragment was isolated by means of agarose gel electrophoresis, electroelution and precipitation
according to known methods.

The isolated fragment containing the ADHI-terminator was
ligated with vector DNA obtained preferably by digestion of
Bluescribe M13+ (see Stratagene, San Diego, CA 92121, USA) with SalI and SphI and after purification of the obtained
vector DNA by means of agarose gel electrophoresis, electroelution and precipitation according to known methods.

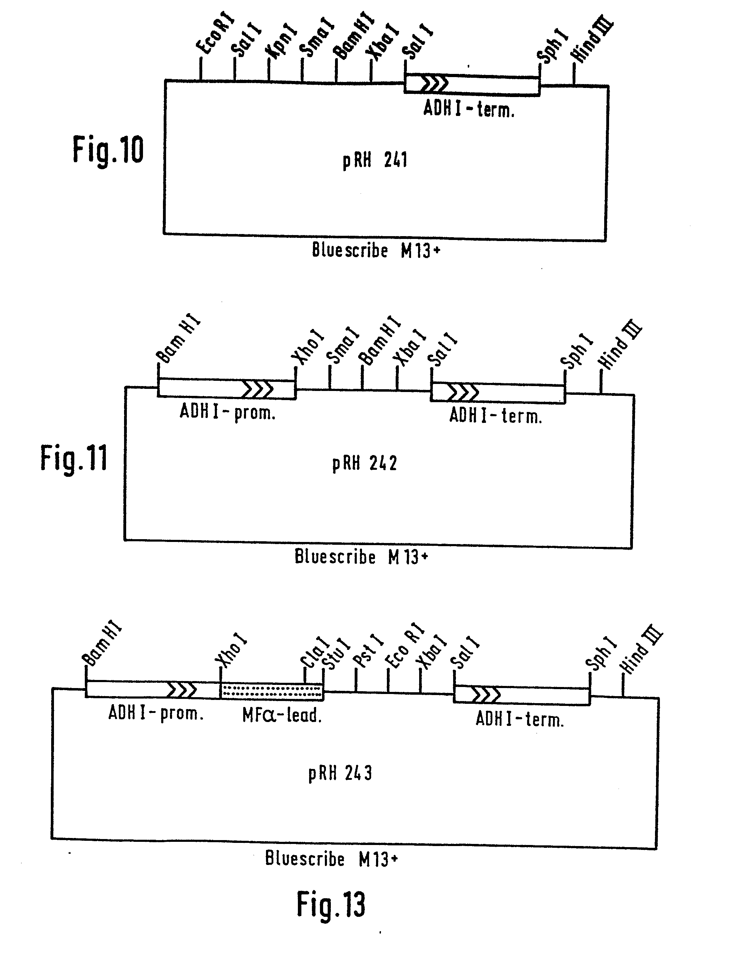

The obtained ligase solution was transformed in E.coli JM101
and the plasmid of one of the ampicillin-resistent colonies
was designated as pRH241 (see Fig. 10) containing an approximately 340 bp long fragment with the ADHI-terminator.


b) The ADHI-promotor can be isolated from a plasmid containing this promotor, for example from the plasmid pY-JDB-
HuIFN-omegal (see Example A and German Patent Application
P 36 35 867.3, filed on October 22, 1986) which is subsequently inserted in a suitable vector, such as the plasmid
pRH241.

The necessary insert was prepared by digestion of the plasmid pY-JDB-HuIFN-omegal with SphI, removing the 3' overhang
using E.coli DNA polymerase I in the presence of dGTP and
recutting with XhoI. After isolating the fragments obtained
by means of agarose gel electrophoresis, by electroelution
and precipitation according to known methods, the blunt end
of the 400 bp long fragment was converted by ligation with
the adaptor pair of the formula


| EBI-410: | 5' | AATTGGAAGGATC | 3' |
|----------|----|--------------|----|
| EBI-429: | 3' | CCTTCCTAG-p | 5' |


and the sticky end by ligation with the adaptor pair of the
formula


| EBI-418: | 5' | p-TCGAGCACGTGGTAC | 3' |
|----------|----|------------------|----|
| EBI-424: | 3' | CGTGCAC | 5' |

The ligations were carried out simultaneously and after purification of the ligation product by means of agarose gel electrophoresis, it was ligated with a suitable linearised vector DNA, preferably with linearised vector DNA obtained by digestion of the plasmid pRH241 with EcoRI and KpnI. The obtained ligase solution was transformed in E.coli, the resulting colonies were checked for the presence of a plasmid with the correct construction according to known methods. One plasmid, designated as plasmid pRH242 (see Fig. 11), was selected.

c) After chemical synthesis of the yeast mating factor α leader peptide (see J. Kurian et al. in Cell 30, 933-943 (1982)), the insert was prepared as follows after synthesis of the following oligodeoxynucleotides

| Name | Sequence |
|---|---|
| MF 1 | TCGAGCCTCATATCAATGAGATTCCCATCTATTTTCACTGCTGTTTTGTT (50 mer) |
| MF 2 | AGCAGCGAACAAAACAGCAGTGAAAATAGATGGGAATCTCATTGATATGA GGC (53 mer) |
| MF 3 | CGCTGCTTCCTCCGCTTTGGCTGCTCCAGTCAACACTACTACTGAAGACG AAACTGCTCAAATTCCAGCT (70 mer) |
| MF 4 | CAGCTTCAGCTGGAATTTGAGCAGTTTCGTCTTCAGTAGTAGTGTTGACT GGAGCAGCCAAAGCGGAGGA (70 mer) |
| MF 5 | GAAGCTGTCATCGGTTACTCTGACTTGGAAGGTGACTTCGACGTTGCT ( 48 mer) |
| MF 6 | GCAAAACAGCAACGTCGAAGTCACCTTCCAAGTCAGAGTAACCGATGA (48 mer) |

MF 7    GTTTTGCCATTCTCCAACTCCACTAACAACGGTTTGTTGTTCATTAAC
        ACTACTATTGCATCGATTGCT (69 mer)


MF 8    CCTTAGCAGCAATCGATGCAATAGTAGTGTTAATGAACAACAAACCGTTG
        TTAGTGGAGTTGGAGAATG (69 mer)


MF 9    GCTAAGGAAGAAGGTGTTTCTTTGGACAAGAGGCCTCTGCAGGAATTCT
        (49 mer)


MF 10   CTAGAGAATTCCTGCAGAGGCCTCTTGTCCAAAGAAACACCTTCTT
        (46 mer)


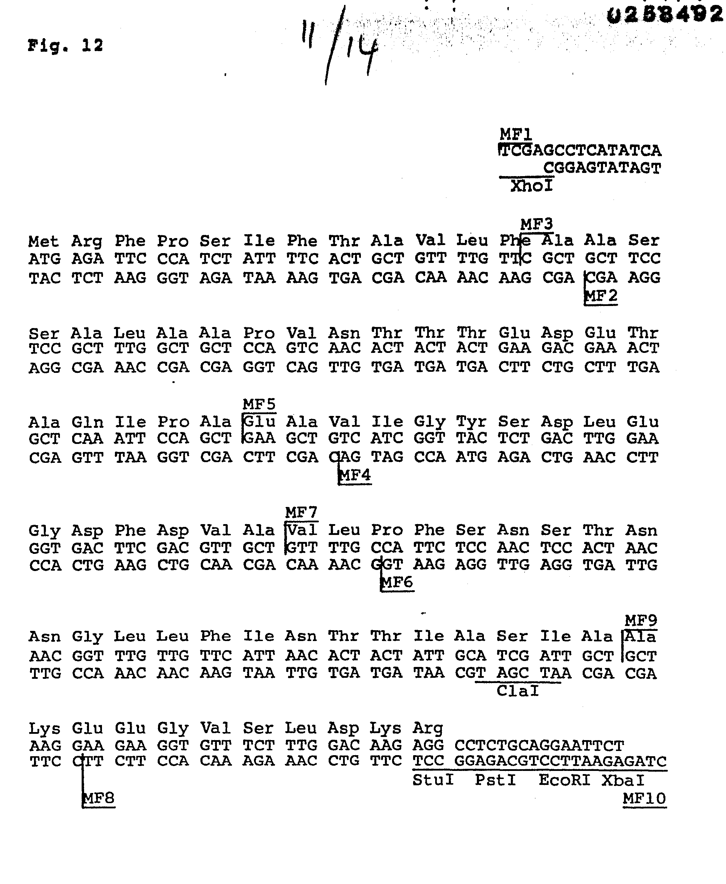

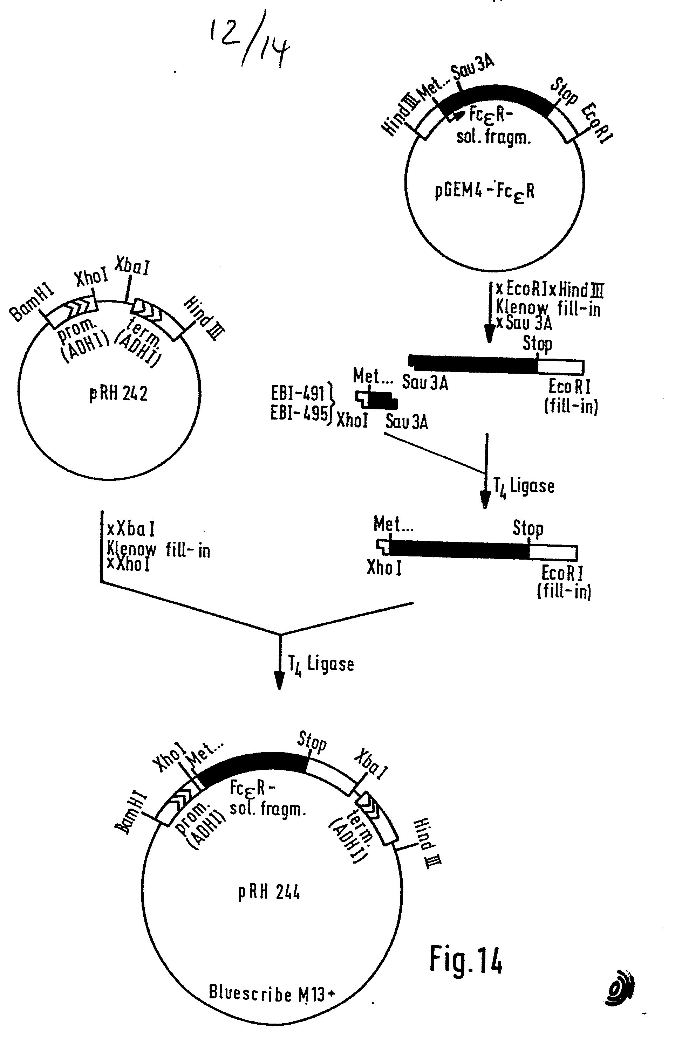

Each of the oligonucleotides MF2 to MF9 were phosphorylated. After stopping the reactions by heating, the following mixtures were prepared: MF1 and MF2; MF3 and MF4; MF5 and MF6; MF7 and MF8; and MF9 and MF10. Then, after heating and cooling, the resulting five solutions were combined and ligated. A linearised vector DNA, preferably obtained by the digestion of pRH242 with XhoI and XbaI after purification by means of electrophoresis, electroelution and precipitation according to known methods, was added to the above solution. This ligation solution was used to transform E.coli JM101, the plasmids of the resulting colonies were checked by digestion with XhoI and XbaI, whereby those plasmids containing an insert of about 290 bp (see Fig. 12) were further characterised by subcloning the insert into M13mp8 and by sequencing according to the dideoxy chain termination method of Sanger (see Proc. Natl. Acad. Sci. $\underline{74}$, 5463-5467 (1977)). One plasmid containing the correct insert was selected and designated as pRH243 (see Fig. 13).


d) The coding sequence for the water-soluble part of human low affinity $Fc_\varepsilon$-receptor was isolated from plasmid pGEM4 by digestion with HindIII and EcoRI, additionally the sticky ends were filled-in using the Klenow fragment of E.coli DNA-Polymerase I and the four deoxynucleosidtriphosphates. The larger fragment was dephosphorylated and purified according to known methods.

Since HindIII cuts the Fc$_\varepsilon$R-cDNA about 50 bp upstream of the first amino acid, the insert is recut with Sau3A. Since this procedure removes not only the 5'upstream region but also the nucleotides for the first 18 N-terminal amino acids of the water-soluble fragment starting at amino acid 150 of the complete Fc$_\varepsilon$-receptor, two oligodeoxynucleotides of formulas

EBI-491:
```
5'   TCGAGCTCATATACAATGG ATG GAA TTG CAA GTT TCC TCT
     GGT TTC GTT TGT AAC ACT TGT CCA GAA AAG TG
```

EBI-495:
```
3'   CGAGTATATGTTACC TAC CTT AAC GTT CAA AGG AGA CCA
     AAG CAA ACA TTG TGA ACA GGT CTT TTC ACC TAG
                                              Sau3A
```

were synthesized to restore the complete gene using the yeast codon usage of the formula

```
                                              Met
                    5'   TCGAGCTCATATACA ATG
                    3'   ____CGAGTATATGT TAC
                         XhoI
```

```
              155                 160                 165
Glu Leu Gln Val Ser Ser Gly Phe Val Cys Asn Thr Cys Pro Glu
GAA TTG CAA GTT TCC TCT GGT TTC GTT TGT AAC ACT TGT CCA GAA
CTT AAC GTT CAA AGG AGA CCA AAG CAA ACA TTG TGA ACA GGT CTT
```

```
Lys Trp
AAG TG          3'
TTC ACC TAG     5'
     Sau3A
```

The prepared oligodeoxynucleotides were annealed, the Sau3A-EcoRI fragment was added and ligated using T4-DNA-ligase. The resulting fragment was purified by electrophoresis, electroelution and precipitation according to known methods.

This insert was ligated with a suitable linearised vector DNA, preferably with the larger fragment obtained by digestion of pRH242 with XbaI (filled-in) and XhoI and by additional purification according to known methods.

The obtained ligation solution was transformed in E.coli JM101, the plasmids of some of the resulting colonies were checked with several restriction enzymes according to known methods. One plasmid containing the correct insert was selected and designated as pRH244 (see Fig. 14).

e) The coding sequence for the water-soluble part of human low affinity Fc$_\varepsilon$-receptor was isolated from plasmid pGEM4-Fc$_\varepsilon$R by digestion with HindIII and EcoRI, additionally the sticky ends were filled in using the Klenow fragment of E.coli DNA-Polymerase I and the four deoxynucleosidtriphosphates. The thus obtained smaller fragment was dephosphorylated and purified according to known methods.

Since HindIII cuts the Fc$_\varepsilon$R-cDNA about 50 bp upstream of the first amino acid, the insert is recut with Sau3A. Since this procedure removes not only the 5'upstream region but also the nucleotides for the first 18 N-terminal amino acids of the water-soluble fragment, two oligodeoxynucleotides of formulas

EBI-430:

   5'  ATGGAATTGCAAGTTTCCTCTGGTTTC ATG GAA TTG CAA GTT TCC
TCT GGT TTC GTT TGT AAC ACT TGT CCA GAA AAG TG

EBI-437:

3'  TACCTTAACGTTCAAAGGAGACCAAAG TAC CTT AAC GTT CAA AGG

AGA CCA AAG CAA ACA TTG TGA ACA GGT CTT TTC ACC TAG      5'

                                         Sau3A

were synthesized to restore the complete gene using the yeast
codon usage of the formula

                                                   Met

                                      5'    ATG

                                      3'    TAC

Glu Leu Gln Val Ser Ser Gly Phe Val Cys Asn Thr Cys Pro Glu
GAA TTG CAA GTT TCC TCT GGT TTC GTT TGT AAC ACT TGT CCA GAA
CTT AAC GTT CAA AGG AGA CCA AAG CAA ACA TTG TGA ACA GGT CTT

Lys Trp
AAG TG       3'
TTC ACC TAG  5'
     Sau3A

Both oligodeoxynucleotides were annealed, the Sau3A-EcoRI
fragment was added and ligated using T4-DNA-ligase. The
resulting fragment was purified by electrophoresis, electroelution and precipitation according to known methods.

This insert was ligated with a suitable linearised vector
DNA, preferably with the larger fragment obtained by digestion of plasmid pRH243 with EcoRI and StuI and by additional purification by means of electrophoresis, electroelution and precipitation according to known methods.

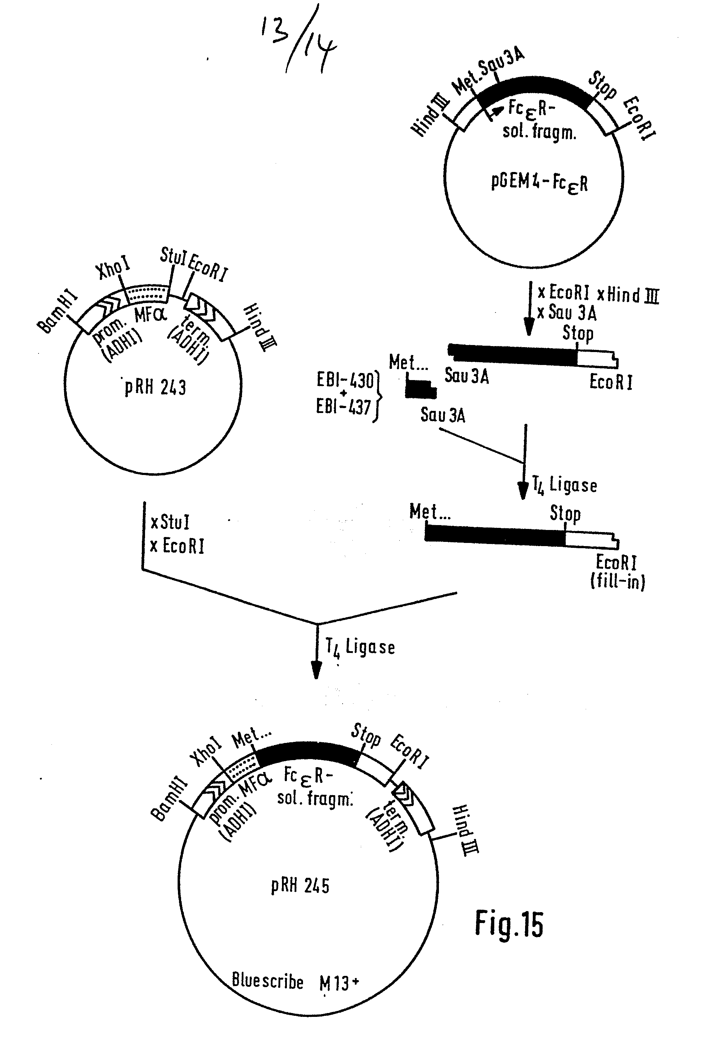

The obtained ligation solution was transformed in E.coli JM101, the plasmids of some of the resulting colonies were checked with several restriction enzymes according to known methods. One plasmid containing the correct insert was selected and designated as pRH245 (see Fig. 15).

f) The expression cassettes of the plasmids pRH244 and pRH245 consisting of ADHI-promotor, MFα-leader gene (only in the case of pRH245), $Fc_\epsilon R$-water-soluble gene and ADHI-terminator were isolated by digestion with HindIII and BamHI and ligated with a yeast plasmid, for example with suitable linearised vector DNA of plasmid pJDB207.

In addition to microorganisms, cultures of cells derived from multicellular organisms may also be used as hosts. In principle, any such cell culture is workable, whether from vertebrate or invertebrate culture. However, interest has been greatest in vertebrate cells, and propagation of vertebrate cells in culture (tissue culture) has become a routine procedure in recent years (Tissue Culture, Academic Press, Kruse and Patterson, Editors (1973)). Examples of such useful host cell lines are VERO and HeLa cells, Chinese hamster ovary (CHO) cell lines, and W138, BHK, COS-7 and MDCK cell lines. Expression vectors for such cells ordinarily include (if necessary) an origin of replication, a promotor located in front of the gene to be expressed, along with any necessary ribosome binding sites, RNA splice sites, polyadenylation site, and transcriptional terminator sequences.

For use in mammalian cells, the control functions on the expression vectors are often provided by viral material. For example, commonly used promotors are derived from polyoma, Adenovirus 2, and most frequently Simian Virus 40 (SV40). The early and late promotors of SV40 are particularly useful because both are obtained easily from the virus as a fragment which also contains the SV40 viral origin of replication (Fiers et al., Nature 273, 113 (1978)). Smaller or larger SV40 fragments may also be used, provided there is included the approximately 250 bp sequence extending from the
Hind III site toward the Bgl I site location in the viral
origin of replication. Further, it is also possible, and
often desirable, to utilize promotor or control sequences
normally associated with the desired gene sequence, provided
such control sequences are compatible with the host cell
systems.

An origin of replication may be provided either by construction of the vector to include an exogenous origin, such as
may be derived from SV40 or other viral (e.g., Polyoma,
Adeno, VSV, BPV, etc.) source, or may be provided by the
host cell chromosomal replication mechanism. If the vector
is integrated into the host cell chromosome, the latter is
often sufficient.

i) The prepared human lower affinity $Fc_\varepsilon$-receptor prepared
according to the invention, preferably the water-soluble
part thereof starting at about amino acid 50 to about 150 of
the whole $Fc_\varepsilon$-receptor, is suitable for the treatment or
prophylaxis of local and allergic reactions induced by expression of IgE and may be incorporated in the suitable pharmaceutical compositions such as solutions or sprays.

The following examples, which are not exhaustive, will
illustrate the invention in greater detail:

General Materials and Methods:

The monoclonal anti-$Fc_\varepsilon$R antibodies 3-5 ($\gamma_1$) and 8-30 ($\mu$)
were produced by hybridization of P3U1 myeloma with spleen
cells from Balb/c mice immunized with RPMI-8866 cells (see
European Patent Application No. 86 110 420.6 of the same
Applicant, filed on July 29, 1986). The 8-30 antibody recognizes the epitope close to IgE binding site of $Fc_\varepsilon$R and

can block binding of IgE to 8866 lymphoblastoid cells. The
3-5 antibody, recognizes a different epitope on $Fc_\varepsilon R$ and can
not block effectively IgE binding to its receptors. These
antibodies precipitate 46 kd and 25 kd polypeptides under
reducing and non reducing conditions. The monoclonal antibodies were purified from ascitis by 50 % saturated ammonium
sulfate precipitation followed by gel filtration using Sepharose 6B (Pharmacia Fine Chemical, Uppsala, Sweden) for IgM
class or ion exchange chromatography using QAE-Sephadex
(Pharmacia Fine Chemical) for IgG1. The polyclonal mouse IgG
was isolated in the same fashion. The anti-mouse IgM-alkaline
phosphatase conjugate was purchased from Tago (Burlingame,
CA).

Reverse phase HPLC was carried out by using a Waters HPLC
system with Hi-Pore, RP-304 (250 x 4.6 mm) (Bio-Rad) column.
The immunoaffinity purified $Fc_\varepsilon R$ was applied to the reverse
phase column equilibrated with water containing 0,1 % trifluoracetic acid and eluted with a linear gradient of acetonitril containing 0,1 % trifluoroacetic acid (TFA). A flow
rate of 0.5 ml/min and a linear gradient (from 0 % to 65 %
for 60 min) of acetonitril was employed. The eluted material
was frozen and lyophilized prior to testing for activity in
ELISA.

## $NaDodSO_4$/PAGE

Crude, immunoaffinity purified and HPLC purified fractions
of Fc R were analyzed by electrophoresis on a 1 % $NaDodSO_4$-
10 % polyacrylamide gel (Fig. 4) and proteins were determined by silver staining using Daiichi-Kagaku silver stain
(Daiichi-Kagaku, Tokyo). To measure $Fc_\varepsilon R$ activity, after
electrophoresis, the gel was cut into 4 mm slices, minced eluted with lysis buffer overnight at room temperature with shaking, the eluted material was collected after centrifugation and tested for activity in ELISA.

The enzyme reactions are performed using standard protocols (see Molecular cloning - a laboratory manual; T. Maniatis et al. (1982), Ed. Cold Spring Harbour) or by following the supplier's instructions. The restriction maps of the described plasmids and the reaction schemes are not drawn to scale.

The oligodeoxynucleotides were synthesized using an Applied Biosystems DNA synthesizer Model 381A and purified by polyacrylamide gel electrophorsis (12 % Acrylamide, 0.6 % Bisacrylamide, 8 M Urea, 1 x TBE buffer), elution and desalting using a Sephadex G25 column.

## Example A

## Preparation of the plasmid pY-JDB-HuIFN-omega1

### a) Production of the yeast-ADHI-promotor fragment
80 µg of the plasmid pES103 in 500 µl are digested with BamHI and XhoI. The approximately 1400 basepair (bp) long promoter fragment is separated from the vector on a 1 % agarose gel, isolated from the gel by electroelution and precipitated. The fragment is suspended in 40 µl TE-buffer (10 mM Tris, 1 mM EDTA, pH 8).

### b) Preparation of vector pJDB 207
10 µg pJDB 207 in 100 µl solution are cut with BamHI and thereby linearised. In order to inhibit re-ligation the 5' terminal phosphate groups are removed by treatment with calf-intestine phosphatase (CIP). The linear form is sepa- rated from any remaining undigested plasmid on a 1 % agarose gel, isolated through electroelution and precipitated. The precipitated vector DNA is dissolved in 20 µl TE-buffer.

c) Expression vector for IFN-omega1
50 µg of plasmid pRHW12 (see EP-A-0.170.204) in 600 µl solution were linearised by cleavage with HindIII. The resulting staggered ends were converted to blunt ends by addition of the Klenow-fragment of DNA-Polymerase I (10 units) and 25 µM each of the four desoxynucleosidetriphosphates, and by incubation at room temperature. The linear form was purified by electrophoresis on agarose gel and subsequent isolation. The fragment was suspended in 50 µl TE-buffer.

In order to obtain ends compatible with the promoter fragment an XhoI-linker is attached to the ends of the linear pRHW12. 3 µl XhoI-linker (0,06 $OD_{250 \text{ nm}}$, Pharmacia P-L, # 27-7758-01, formula d[CCTCGAGG]) in 20 µl solution are phosphorylated using 5 units of T4-polynucleotidkinase and rATP. After inactivation of the enzyme by heating at 70°C for 10 minutes 5 µl of this solution are combined with 10 µl of linear pRHW12 and in total 20 µl of reaction solution subjected to ligation using T4-DNA ligase (for 16 hours at 14°C). The ligase is then inactivated by heating at 70°C for 10 minutes and the reaction volume brought to 150 µl with 1 x medium buffer (10 mM Tris, pH 7,5, 50 mM NaCl, 10 mM $MgCl_2$).

The XhoI specific 5' sticky ends are generated by treating with 100 units of XhoI. The linear pRHW12 having XhoI ends is purified by electrophoresis on agarose gel and electroeluted from the gel. Before the precipitation 5 µl of promoter fragment (from section a) are added. After the precipitation the DNA is suspended in 14,5 µl of TE-buffer, Ligase buffer and 5 units of T4-DNA ligase are added and ligation carried out for 16 hours at 14°C. After inactivation of the enzyme the volume is brought to 200 µl and the DNA cleaved using BamHI. The DNA is obtained by purification on agarose gel and is dissolved in 20 µl TE-buffer.

## d) Ligation of the fragments

The final expression vector is obtained by treating 5 µl of the BamHI fragments (Promoter and IFN-omegal-gene) with 1 µl of the linearised pJDB207 vector (yeast 2 µ Terminator and yeast 2 replication origin, Leu2 Marker, E.coli replication origin, Ampicillin resistence gene) in 10 µl solution in the presence of 1 unit of T4-DNA ligase.

## e) Transformation

10 µl of competent E.coli HB101 cells were transformed by the addition of 5 µl ligase solution and plated on LB-agar containing 100 µg/ml Ampicillin. 12 of the resulting clones were selected and the plasmids isolated. After cutting of the plasmids with various restriction enzymes and electrophoresis on agarose gel a plasmid was chosen which demonstrated the correct construction; it was designated pY-JDB-HuIFN-omegal.

## Example B

## Construction of expression plasmid pRH 100

7 µg of plasmid pER 103 (see Eva Dworkin-Rastl et al., Gene 21, 237-248 (1983) and EP-A-0.115.613)) were linearised in 50 µl of reaction medium with the restriction endonuclease HindIII. After incubation for 1 hour at 37°C, 50 µl of 2 x CIP buffer were added (2 x CIP buffer = 20 mM Tris, pH=9.2, 0.2 mM EDTA). After the addition of 2 units of alkaline phosphatase from calf intestine (CIP) the 5' terminal phosphate residues were removed; incubation was carried out for 30 minutes ab 45°C. The reaction was stopped by the addition of 4 µl of 0.5 EDTA solution and the addition of 10 µl of 1M Tris, pH=8.0 solution. The proteins were removed
by extracting twice with phenol and once with phenol/chloroform. The DNA was precipitated from the aqueous phase after
the addition of 0.1 vol 3M sodium acetate solution pH=5.5
and 250 µl of ethanol and the DNA precipitate after being
centrifuged was washed once with 70 % ethanol solution. The
DNA was dried and the pellet was then dissolved in 20 µl of
TE buffer (10 mM Tris pH=8.01, 1 mM EDTA).

1 µl batches of the synthetic oligodeoxynucleotides
d(AGCTTAAAGATGAGCT) and d(CATCTTTA) were phosphorylated in
10 µl of reaction solution with the addition of 10 units of
T4-PNK (polynucleotide kinase) and 1 mM rATP. The reaction
took place at 37°C and lasted 45 minutes. The reaction was
stopped by heating at 70°C for 10 minutes.

5 µl of the plasmid solution and the phosphorylated oligonucleotide were mixed together and heated to 70°C for 5 minutes. The solution was then cooled to 0°C and 2 µl of 10 x ligase buffer (500 mM Tris, pH=7.5), 100 mM $MgCl_2$ 200 mM DDT
(dithiothreitol), 1 mM rATP, 500 µg/ml BSA (bovine serum albumin), 2 µl of water and 10 units of T4-DNA ligase were
added. The reaction lasted 40 hours and was carried out at
4°C. It was stopped by heating at 70°C for 10 minutes.

2 µl of this ligase reaction were digested in a total of
30 µl of solution with 10 units of the restriction endonuclease SacI (New England Biolabs) for 3 hours at 37°C. The reaction was stopped by heating to 70°C for 10 minutes. 5 µl of
this reaction mixture were ligated in a total of 30 µl by
adding 10 units of T4-PNK at 14°C for 16 hours.

200 µl of competent E.coli HB101 were mixed with 10 µl of
this ligase reaction. The bacteria were kept on ice for 45
minutes and then heated to 42°C for 2 minutes in order to
allow DNA uptake. The bacterial suspension was further incubated at 0°C for 10 minutes. Finally the transformed bacteria were plated on LB agar containing 50 µl/ml of ampicillin.

12 of the bacterial colonies were chosen at random and the plasmids from them were isolated at a microscale (see Birnboim et al. in Nucl. Acids Res. 7, 1513-1523 (1979)). The resulting DNA was cut with the restriction endonuclease SacI and the DNA was separated on an agarose gel (1 %, 1 x TBE buffer). The migration of the DNA as a linear 4.400 pb molecule confirmed that a SacI recognition site had been inserted into the plasmid. One of these plasmids was randomly selected. E.coli HB101 was again transformed with the DNA from the corresponding mini preparation. From the resulting transformed bacteria, a colony was selected and grown at a larger scale. The plasmid isolated therefrom was cut with the restriction endonucleases EcoRI and BamHI, the DNA was separated on a 1 % agarose gel and the smaller fragment was isolated from the gel by electroelution. This EcoRI-BamHI DNA fragment, about 460 bp long, was sequenced according to Sanger (see F. Sanger et al. in Proc. Natl. Acad. Sci. 74, 5463-5467 (1977)). The plasmid analysed in this way was designated pRH 100.

Example 1

a) Isolation of crude $Fc_\varepsilon R$ from culture supernatant

RPMI-8866 cells were cultured in RPMI1640 medium supplemented with 10 % fetal calf serum and antibiotics (100 units/ml of penicillin and 100 µg/ml of Streptomycin) at a density of $1 \times 10^6$ cells/ml and a cell viability of 95-99 % in Spinner bottles. The cells were harvested after 15 min centrifugation at 5,000 rpm washed 3 times with Hank's BSS and cultured at the same density in serum-free medium for 48 hours in Spinner bottles. The culture supernatant was collected and supplemented with phenylmethyl sulfonylfluoride (PMSF) (1 mM), 0,02 % sodium azide ($NaN_3$), and 200 units/ml of aprotenin. The culture supernatants were stored at 4°C during concentration.

Concentration of RMPI-8866 culture sups was carried out by an Amicon filter system using a DIAFLO, YM10-filter. The 200-300 times concentrated material was then centrifuged at 85,000 x G for 40 min at 4°C in order to remove insoluble material, whereby crude $Fc_\varepsilon R$ preparation was obtained.

b) <u>Isolation of $Fc_\varepsilon R$ from cell lysates</u>

RMPI-8866 cells (2 x $10^9$ cells) were washed 4 times with PBS and lysed in 10 ml of lysis buffer (PBS containing 0,5 % Nonindet P-40 (NP-40) and 1mM PMSF for 45 min on ice with periodic vortexing. An additional 10 ml of lysis buffer was added and the lysis continued for an additional 30 min on ice. The lysate was centrifuged at 37.000 rpm for 45 min at 4°C. The lipid layer was carefully removed and the supernatant collected and stored at -70°C.

Example 2

<u>Immunoaffinity Purification</u>

Culture supernatant concentrate (see Example 1a) equivalent to 5-10 liters of culture were sequentially adsorbed on 2 ml of BSA-Sepharose, human transferrin-Sepharose and normal mouse Ig (NMIg)-Sepharose gels for one hour each at 4°C with rotation. The effluent collected from the last immunoaffinity gel was then applied on 2 ml of anti-$Fc_\varepsilon R$(3-5) coupled to Sepharose. Immunoadsorption was allowed to proceed for between 4-16 hours at 4°C with rotation. The gel was poured into a column, the effluent collected and the gel washed sequentially with 150 ml of buffer A (Tris-HCL, 10 mM, pH 7.5, NaCl, 0.5 M, NP-40, 0.5 %), 150 ml of buffer B (Tris-HCl, 10 mM, pH 7.5, NP.40 0,1 %), 150 ml of buffer C (Tris-HCl, 10 mM, pH 7.5) and eluted with 25 ml of 2.5 % acetic acid (v/v) and immediately neutralized in Tris-HCl,2 M, pH 8.0. The material was stored at -80°C if not used immediately for further purification utilizing HPLC. - Then, the eluate was fractionated by a reverse phase HPLC on a C4 column utilizing a linear gradient of 0-65% acetonitril containing 0,1% trifluoroacetic acid.

Example 3

Enzyme Linked Immunosorbent Assay (ELISA)

The Fc$_\varepsilon$R activity was measured by its ability to bind to the monoclonal antibodies 3-5 and 8-30 and monitored utilizing a double antibody enzyme-linked immunosorbent assay (ELISA). 96 well microtiter plates were initially coated with the monoclonal antibody 3-5 100 µl/well (10 µg/ml) in coating buffer (NaHCO$_3$ 0.1 M, pH 9.6), and incubated overnight at 4°C. The plates were then washed 4 times with rinse buffer, i.e. Dulbecco's, phosphate buffer pH containing 0,05 % Tween 20, followed by the addition of 100 µl test sample diluted with diluent buffer (Tris-HCl 0.05 M, pH 8.1, MgCl$_2$ 1mM, NaCl 0,15 M, Tween 20 0.05 % (v/v), NaN$_3$ 0.02 %, BSA 1 %). The microtiter plates were incubated for 2 hours at room temperature, and washed 4 times with rinse buffer, followed by the addition of 100 µl of a pretitrated and diluted goat-anti-mouse IgM-alkaline phosphatase conjugates. The plates were incubated for two hours at room temperature and washed 4 times with rinse buffer. In the final step 200 µl of substrate, p-phenyl phosphate disodium (1 mg/ml) in substrate buffer (NaHCO$_3$ 0.05 M, pH 9.8, MgCl$_2$ x 6 H$_2$0, 10 mM) was added and the colorimteric reaction measured every 30 min for two hours at 405 and 620 nm.

## Example 4

## Hydrolysis of Fc$_\varepsilon$-receptor by means of lysylendopeptidase and Separation of Peptides

The purified Fc$_\varepsilon$R was digested with Achromobacter lyticus lysylendopeptidase in 50 mM Tris-HCl buffer, pH 9.5 for 6 hours at 35°C at an enzyme-to-substrate ratio of 1:500 (W/W). The lyophilized peptide fragments were purified by HPLC.

## Separation of Peptides by Reverse Phase HPLC

Separation of peptides was performed by HPLC using a C4 column on a Hitachi 655 liquid chromatograph equipped with a 655-60 gradient processor and a Rheodyne Sample injector with a 100 μl sample loop. The elution of peptides was carried out with a linear gradient of 2-propanol: acetonitril = 7:3 (v/v) from 0-35 % for 1 hour in 0.1 % trifluoracetic acid and a flow rate of 1.0 ml/min.. The fractionated peptides were manually collected by monitoring the absorbance at 215 nm.

## Amino Acid Analysis and Sequence Determination

The amino acid analyses were carried out with a Hitachi 835-5 amino acid analyzer after hydrolysis of the peptide fragments in 5.7 HCl at 110°C in evacuated, sealed tubes for 22-24 hours. Automated Edman degradation was performed with a 470 A protein sequencer (Applied Biosystems, Inc. CA) using a standard program for sequencing. The phenylthiohydantoin (PTH)-amino acids were determined by reverse phase HPLC with isooratic elution (see Tsunasawa et al. in J. Biochem. 97, 701-704 (1985)).

The following amino acid sequences were detected:

Met-Glu-Leu-Gln-Val-Ser-Ser-Gly-Phe-Val-,

Gly-Glu-Phe-Ile-Trp-Val-Asp-Gly-Ser-His-Val-Asp-Tyr-Ser-Asn-Trp-Ala-Pro-Gly-Glu-Pro-Thr-,

Lys-His-Ala-Ser-His-Thr-Gly-Ser-Trp-Ile-Gly-Leu-Arg-Asn-Leu-Asp-Leu-Lys- and

Lys-Trp-Ile-Asn-Phe-Gln-.

Example 5

Preparation of the oligonucleotide of formula

$$3'-TT^T_C \; ACC \; TA^T_A G \; TT^A_G \; AA^A_G \; GT \; -5'$$

The oligonucleotide was synthesized using an ADI-synthesizer at the 0,2 µMol level.

The resulting oligonucleotide was demethylated with thiophenol/triethylamin/dioxan (1:2:2) at room temperature within 90 minutes and washed with methanol (10 x 1 ml of methanol).

After removing the demethylated oligonucleotide from controlled pore glass (CPG) and splitting off the protective group with concentrated ammonia within 1 hour at room temperature and 16 hours at 55°C, the oligonucleotide was dried by means of Speedvac ®.

Further purification was carried out over 20 % of acrylamide/8 Mol of urea gel with TBE-buffer (10,9 g of tris (hydroxymethyl)aminomethane, 5,5 g of boric acid and 9,3 g of Ethylenedinitrilo-tetraacetic acid disodium salt in 1 1).

The subsequent gel-electrophorese (40 cm x 25 cm x 0,1 cm) was carried out at 50 watt. The 17-mer band was cut out, eluted with water and desalted on a 0,9 x 13 cm Sephadex G 25 medium column in water.

The fractions containing the 17-mer were pooled and dried. Yield: 7,7 $OD_{260}$ ( $\sim$285 µg)

Example 6

Establishment of $Fc_\epsilon R^+$ L cell transformants

One million $Ltk^-$ cells were co-transfected with 150 ng Herpes simplex virus derived tk gene and 20 µg high molecu-lar weight genomic DNA from RPMI-8866 cells (see Wigler et al. in Cell 16, 777-785 (1978)). Cells were selected after being kept in HAT medium for 10 days. L cells showing resistence to HAT were collected, stained with biotinated anti-$Fc_\epsilon R$ (8-30) and fluorescein isothiocyanate (FIIC) conjugated avidin and sorted by FACS. Several cycles of sorting were carried out for each transfection. Two transformant lines, L-V-8-90 and L-VI-8-30 were obtained from independent transfections.

Example 7

Cloning of the $Fc_\epsilon R$ cDNA

a. Probe I: Total RNA was isolated from the $Fc_\epsilon R^+$ L cell transformant L-V-8-30 by the guanidium/cesium chloride method (see Chirgwin et al. in Biochemistry 18, 5294-5299 (1979)). Poly(A)$^+$ RNA was prepared by oligo dT cellulose chromatography. Radio-labeled cDNA was synthesized from poly(A)$^+$ RNA by using $^{32}$P-dCTT (see Davis et al. in Proc. Natl. Acad. Sci. USA 81, 2194-2198 (1984)). The labelled cDNA was annealed with an excess of poly(A)$^+$ RNA of untransformed Ltk$^-$ cells and applied on hydroxyapatite column. The single-stranded cDNA was collected and used as probe I.

b. Probe II: The 17-mer oligonucleotides complementary to the mRNA sequence encoding for the amino acid fragment Lys-Trp-Ile-Asn-Phe-Gln, containing a mixture of 24 possible sequences, were radiolabeled with γ-$^{32}$P-ATP by T4 polynu-cleotide kinase.

Double-stranded cDNA was synthesized from poly(A)$^+$ RNA derived from RPMI-8866 cells using Amersham cDNA synthesis system (Amersham, UK) (see Gubler and Hoffman in Gene 25, 263-269 (1983)). After treatment with EcoRI methylase and T4 DNA polymerase, the double stranded cDNA longer than 1,000 bp was cloned into the EcoRI site of λgt10 using EcoRI linkers and packaged in vitro using Gigapack (Vector cloning systems). Two sets of replica filters of phage plaques were made. One set of filters were hybridized with $^{32}$P-labeled 17-base long oligonucleotides (Probe II) (see Wood et al. in Proc. Natl. Acad. Sci. USA 83, 1585-1588 (1985)). Another set of filters were hybridized with $^{32}$P-labeled subtracted cDNA specific to Fc$_\varepsilon$R positive L cells (Probe I) overnight in 6xSSC at 68°C and washed with 0.1xSSC and 0,1 % SDS for 2 hours. The plaques which hybridized with both probes were isolated.

## Example 8

## Expression of $Fc_\varepsilon R$ cDNA

a) For expression of $Fc_\varepsilon R$ cDNA in pGEM4 using SP6 promotor, mRNA was synthesized with SP6 RNA polymerase using BamHI digested $pFc_\varepsilon R$-1 as a template (see Melton et al. in Nucl. Acids. Res. 12, 7035-7056 (1984)). 10 ng mRNA were injected into one oocyte and as a negative control, murine BSF-1 mRNA was similarly prepared and injected into another oocyte. After 2 days incubation in Barth's medium at 20°C, the oocytes were lysed in 50 µl lysis buffer (10 mM Tris-HCl, pH 7.5, 0.5 M NaCl, 0,5 % NP40). The oocyte lysates were tested for $Fc_\varepsilon R$ activity using an ELISA method consisting of a sandwich technique and using two anti $Fc_\varepsilon R$ antibodies, 3-5 and 8-30. As a positive control, concentrated culture supernatant from RPMI-8866 cells was employed.

b) For expression of $Fc_\varepsilon R$ cDNA in Cos-7 cells, the insert of $pFc_\varepsilon R$-1 was cloned into the EcoRI site of pDE2 vector (see Japanese Patent Publication 1986/88879 from May 7, 1986). Cos-7 cells ($5 \times 10^5$) were seeded onto 60 mm plates one day prior to transfection. Transfection was carried out with 2 µg of plasmid DNA in 1 ml of 25 mM Tris-HCl (pH 7.5), 137 mM NaCl, 5 mM KCl, 0.6 mM $Na_2HPO_4$, 0.5 mM $MgCl_2$, 0.7 mM $CaCl_2$ and 500 µg of DEAE-dextran (Pharmacia Fine Chemical). After 1 hour incubation at 37°C, the solution was replaced with DMEM containing 10 % FCS and 150 µM chloroquine, incubated at 37°C for 3 hours and then replaced with fresh DMEM containing 10 % FCS. 48 hours later cells were stained with phycocyanin conjugated anti-$Fc_\varepsilon R$ antibody (3-5) and biotinated IgE, developed with FITC-avidin and analyzed by a dual laser FACS (see Kikutani et al. in J. Exp. Med. In press (1986)).

Example 9


Determination of the nucleotide sequence of the Fc$_\varepsilon$R cDNA

---

The insert of pFc$_\varepsilon$R-1 was digested with HindIII and PvuII restriction enzymes. Digested DNA was subcloned in M13 and sequenced (see Sanger et al. in Proc. Natl. Acad. Sci. USA 74, 5463-5467 (1977)) and confirmed by the procedure of Maxam and Gilbert (see Proc. Natl. Acad. Sci. USA 74, 560-564 (1977)).


Example 10


Northern blot analysis of Fc$_\varepsilon$R mRNA

---

Three micrograms of poly(A)$^+$ RNA from various cells were separated on 1 % agarose gel, transferred to nitrocellulose papers and hybridized with a nick translated pFc$_\varepsilon$R-1 insert (see Thomas et al. in Proc. Natl. Acad. Sci. USA 77, 5201-5205 (1980)). For BSF-1 induction, one hundred million B or T cells were cultured with or without 10 µg/ml IgE and 50 µ/ml recombinant human BSF-1 (see Yokota et al. in Proc. Natl. Acad. Sci. USA in press (1986)) for 24 hours and 10 µg of total RNA was extracted and analyzed by Northern blotting as described above.


Example 11


Expression of the water-soluble part of human low affinity Fc$_\varepsilon$-receptor in yeast

---

a) Preparation of plasmid pRH 241 containing the yeast ADHI-terminator:

## Vector preparation

10 µg Bluescribe M13+ (Stratagene, San Diego, CA92121, USA)
were doubly digested with 20 units each of SalI and SphI.
The reaction was terminated by adding 1/25 vol of 0,5 M EDTA
(ethylenedinitrilotetraacetic acid disodium salt) and heating at 70°C for 10 minutes. The cut vector was purified by
agarose gel electrophoresis (1 % agarose, 1 x TBE-buffer
(1 x TBE-buffer: 6.05 g/l Tris(hydroxylmethylaminomethane),
3.1 g/l boric acid, 0.37 g/l EDTA); containing 0,5 µg/ml
Ethidiumbromide; electrophoresis at 4 V/cm). After localizing the DNA band using UV light (254 nm) the DNA was
electroeluted using DE81 paper and purified by a final
ethanol precipitation. The DNA was dissolved in 10 µl TE
(10 mM Tris, pH=8.0, 1 mM EDTA).

## Insert preparation

10 µg pWS214S4 were digested using 20 units each of SphI and
SalI. The smaller fragment containing the ADHI-terminator
was isolated via agarose gel electrophoresis, electroelution
and precipitation. The DNA was finally dissolved in 10 µl TE.

1 µl vector DNA and 1 µl insert DNA were ligated in 10 µl
solution using T4-DNA ligase. 3 µl of the ligation solution
were used to transform E.coli JM101 (supE, thi, del(lac-
proAB), F'[traD36, proAB, laclq, lacZ-delM15], lambda mi-
nus). Some of the resulting colonies after Ampicillin selection were grown up at a 1 ml scale. The plasmids were isolated (see Birnboim H. et al. in Nucl. Acids Res. 7, 1513
(1979)) and digested with SphI and SalI. The fragments were
separated on an agarose gel. The presence of the ADHI-terminator fragment was confirmed by a appr. 340 bp (base pair)
DNA fragment. One of the plasmids was selected for further
use and designated as pRH 241 (restriction map: see Fig. 10).

0258492

b) Preparation of plasmid pRH 242 containing the yeast ADHI-promotor and the yeast ADHI-terminator (see J. L. Bennetzen et al. in J. Biol. Chem. 257, 3018-3025 (1982)):

Vector preparation

10 µg of pRH 241 were doubly digested with EcoRI and KpnI. The vector part was freed from the small fragment by agarose gel electrophoresis, electroelution and precipitation. It was finally dissolved in 10 µl TE.

Insert preparation

10 µg of the plasmid pY-JDB-Hu-IFN-omega1 (see example A and German Patent Application P 36 35 867.3, filed on October 22, 1986) were cut with SphI. The 3'overhang was removed adding 5 units of E.coli DNA polymerase I in the presence of dGTP. The DNA was futher cut with XhoI and the resulting fragments isolated via agarose gel electrophoresis, electroelution and precipitation. The blunt end of the 400 bp long fragment containing the ADHI promotor was converted by ligation of the adaptor pair:

| EBI-410: | 5' | AATTGGAAGGATC | 3' |
| EBI-429: | 3' | CCTTCCTAG-p | 5' |

The sticky end of the restriction fragment was converted using the adaptor pair:

| EBI-418: | 5' | p-TCGAGCACGTGGTAC | 3' |
| EBI-424: | 3' | CGTGCAC | 5' |

After the simultaneous ligation of both adaptors the ADHI-promotor fragment was again purified via agarose gel electrophoresis. The DNA was dissolved in 5 µl TE.

1 µl vector and 5 µl insert were ligated in a total of 10 µl solution using T4-DNA ligase. By ligating the insert into the vector the EcoRI and the KpnI sites are destroyed. 3 µl of the ligation solution were used to transform E.coli JM 101. Several colonies were checked at a microscale for the presence of a plasmid with the desired construction by restricting the plasmids with several restriction enzymes. One plasmid was selected and designated as pRH 242 (restriction map: see Fig. 11).

c) Preparation of plasmid pRH 243 containing the yeast ADHI-promotor, a gene coding for the yeast mating factor α (MFα) leader peptide (see J.Kurjan et al. in Cell 30, 933-943 (1982)), a multicloning site and the yeast ADHI-terminator:

The MFα leader peptide gene was chemically synthesized by using the yeast codon usage (see J.L.Benetzen et al. in J. Biol. Chem. 257, 3026-3031 (1982)).

Vector preparation

1 µg pRH242 was doubly digested with XhoI and XbaI. The vector fragment was purified by agarose gel electrophoresis, electroelution and precipitation. The DNA was dissolved in 30 µl TE.

Insert preparation

Using an Applied Biosystems 381A DNA Synthesizer a set of 10 oligodeoxynucleotides was prepared:

Name                          Sequence

MF 1    TCGAGCCTCATATCAATGAGATTCCCATCTATTTTCACTGCTGTTTTGTT
        (50 mer)

MF 2    AGCAGCGAACAAAACAGCAGTGAAAATAGATGGGAATCTCATTGATATGA
        GGC (53 mer)

MF 3    CGCTGCTTCCTCCGCTTTGGCTGCTCCAGTCAACACTACTACTGAAGACG
AAACTGCTCAAATTCCAGCT (70 mer)

MF 4    CAGCTTCAGCTGGAATTTGAGCAGTTTCGTCTTCAGTAGTAGTGTTGACT
GGAGCAGCCAAAGCGGAGGA (70 mer)

MF 5    GAAGCTGTCATCGGTTACTCTGACTTGGAAGGTGACTTCGACGTTGCT
(48 mer)

MF 6    GCAAAACAGCAACGTCGAAGTCACCTTCCAAGTCAGAGTAACCGATGA
(48 mer)

MF 7    GTTTTGCCATTCTCCAACTCCACTAACAACGGTTTGTTGTTCATTAAC
ACTACTATTGCATCGATTGCT (69 mer)

MF 8    CCTTAGCAGCAATCGATGCAATAGTAGTGTTAATGAACAACAAACCGTTG
TTAGTGGAGTTGGAGAATG (69 mer)

MF 9    GCTAAGGAAGAAGGTGTTTCTTTGGACAAGAGGCCTCTGCAGGAATTCT
(49 mer)

MF 10   CTAGAGAATTCCTGCAGAGGCCTCTTGTCCAAAGAAACACCTTCTT
(46 mer)

60 pMol of each oligodeoxynucleotide except MF 1 and MF 10
were phosphorylated in 5 µl solution using T4-Polynucleoti-
dekinase (PNK). The reactions were stopped by heating the
samples at 100°C for 10 minutes. 5 µl MF 1 (60 pMol) and the
MF 2 solution were combined, as well as the solutions of MF 3
with MF 4; MF 5 with MF 6; MF 7 with MF 8; and 5 µl of MF 10
were added to the solution of MF 9. The combined solutions
were heated again at 100°C and slowly cooled down to room
temperature. After this annealing step the five solutions
were combined, 20 units of T4-ligase were added and the ligation was performed at 14°C for 16 hours.

Finally 0,5 µl of vector DNA and 7 µl of the above ligation reaction were combined and ligated using 5 units T4-ligase. E.coli JM101 was transformed with 3 µl of this ligation solution. The plasmids from several colonies were isolated, doubly restricted with XhoI and XbaI and purified with agarose gelelectrophoresis. Those plasmids containing an insert of the expected size (290 bp) were further characterized by subcloning the insert into M13mp8 and sequencing using the Sanger dideoxy chain termination method (see Sanger F. et al. in Proc. Natl. Acad. Sci. 74, 5463-5467 (1977)). One plasmid containing the expected insert (see Fig. 12) was designated as pRH 243 (restriction map: see Fig. 13).

d) Preparation of plasmid pRH 244 containing the ADHI-promotor, the coding sequence for the $Fc_\epsilon R$-water-soluble fragment and the ADHI-terminator (see fig. 15):

Insert preparation

20 µg $pGEM4-Fc_\epsilon R$ (plasmid pGEM (Promega Biotec, Madison, WI53711, USA) containing the larger HindIII-EcoRI fragment of the $Fc_\epsilon R$-cDNA) were cut with 40 units each of EcoRI and HindIII and the sticky ends filled in using the Klenow fragment of E.coli DNA-polymerase I and the four deoxynucleosidetriphosphates (dXTP's). The DNA was dephosphorylated using 10 units of calf intestine phosphatase (CIP, Boehringer Mannheim), freed from protein by two phenol/chloroform extractions and separated on an agarose gel. After electroelution and precipitation the fragment containing the coding region of the $Fc_\epsilon R$-water-soluble fragment dissolved in 10 µl TE.

Since HindIII cuts the $Fc_\epsilon R$ cDNA about 50 bp upstream of the first aminoacid of the soluble fragment (Met-150), the insert of $pGEM4-Fc_\epsilon R$ is recut with Sau3A. This procedure not only removes the 5'upstream region but also the nucleotides coding for the first 18 N-terminal amino acids of the soluble fragment. Two linker oligodeoxynucleotides of the formulas

EBI-491:

  5'   TCGAGCTCATATACAATGG ATG GAA TTG CAA GTT TCC TCT
       GGT TTC GTT TGT AAC ACT TGT CCA GAA AAG TG

EBI-495:

  3'   CGAGTATATGTTACC TAC CTT AAC GTT CAA AGG AGA CCA
       AAG CAA ACA TTG TGA ACA GGT CTT TTC ACC TAG
                                                  Sau3A

were synthesized to restore the complete gene using the yeast codon usage of the formula

```
                                                     Met
                           5'   TCGAGCTCATATACA ATG
                           3'   ____CGAGTATATGT TAC
                           ·    XhoI
```

```
                 155                  160                  165
Glu Leu Gln Val Ser Ser Gly Phe Val Cys Asn Thr Cys Pro Glu
GAA TTG CAA GTT TCC TCT GGT TTC GTT TGT AAC ACT TGT CCA GAA
CTT AAC GTT CAA AGG AGA CCA AAG CAA ACA TTG TGA ACA GGT CTT
```

```
Lys Trp
AAG TG          3'
TTC ACC TAG     5'
     Sau3A
```

25 pMol each of the oligodeoxynucleotides were annealed to each other and added to approximately 3 µg of the Sau3A (5'Phosphat)-EcoRI (filled-in, dephosphorylated) fragment and ligated in a total of 20 µl using T4-DNA ligase. The resulting XhoI-(EcoRI)-fragment was purified by agarose gel
electrophoresis, electroelution and precipitation. It was
dissolved in 20 µl TE.

Vector preparation

10 µl pRH 242 were cut with XbaI and the ends made blunt by
the Klenow fill in reaction. The DNA was recut with XhoI and
the large fragment isolated via agarose gel electrophoresis,
electroelution and precipitation. It was dissolved in 100 µl
TE.

1 µl vector and 1 µl of insert were ligated in a total of
10 µl using T4-DNA ligase (The ligation of a filled-in EcoRI
site onto a filled in XbaI site restores both the EcoRI and
the XbaI site). 3 µl of the ligation reaction were used to
transform E.coli JM101. Plasmids from several colonies were
isolated and checked for the presence of the $Fc_\varepsilon$ R-water-so-
luble fragment gene using several restriction enzymes. One of
the plasmids was further selected and the XhoI-XbaI fragment
partially sequenced to confirm the correct junction between
the ADHI-promotor and the human gene. This plasmid was designated as pRH 244 (see Fig. 14).

e) Preparation of plasmid pRH 245 containing the yeast ADHI-
promotor, the yeast mating factor α leader gene, the gene
for the $Fc_\varepsilon$ F-water-soluble fragment and the yeast ADHI-terminator (see Fig. 15):

Vector preparation

10 µg pRH 243 are doubly digested with EcoRI and StuI. The
large fragment was purified via agarose gel electrophoresis,
electroelution and precipitation. It was finally dissolved
in 100 µl TE.

## Insert preparation

20 µg of pGEM4-Fc$_\varepsilon$R were doubly digested with EcoRI and HindIII and dephosphorylated. The insert was recut with Sau3A and the larger fragment isolated. To restore the complete coding region for the soluble fragment of the formula

```
                                                    Met
                                            5'   ATG
                                            3'   TAC
```

```
Glu Leu Gln Val Ser Ser Gly Phe Val Cys Asn Thr Cys Pro Glu
GAA TTG CAA GTT TCC TCT GGT TTC GTT TGT AAC ACT TGT CCA GAA
CTT AAC GTT CAA AGG AGA CCA AAG CAA ACA TTG TGA ACA GGT CTT
```

```
Lys Trp
AAG TG           3'
TTC ACC TAG      5'
       Sau3A
```

two oligodeoxynucleotides of the formula

EBI-430:

```
  5'  ATGGAATTGCAAGTTTCCTCTGGTTTC ATG GAA TTG CAA GTT TCC
  TCT GGT TTC GTT TGT AAC ACT TGT CCA GAA AAG TG
```

EBI-437:

```
  3'  TACCTTAACGTTCAAAGGAGACCAAAG TAC CTT AAC GTT CAA AGG
  AGA CCA AAG CAA ACA TTG TGA ACA GGT CTT TTC ACC TAG      5'
                                                    Sau3A
```

were synthesized.

25 pMol of each oligodeoxynucleotide were annealed to each other and added to 3 µg of the Sau3A-EcoRI fragment. Ligation was performed in 20 µl solution using T4-DNA ligase.

The resulting DNA was purified by agarose gel electrophoresis, electroelution and precipitation. The DNA was dissolved in 20 µl TE.

1 µl vector fragment and 1 µl insert fragment were ligated in a total of 10 µl using T4-DNA ligase. 3 µl of the ligation reaction were used to transform E.coli JM101. Plasmids from several colonies were isolated and checked for the presence of the $Fc_\varepsilon R$-water-soluble fragment gene using several restriction enzymes. One of the plasmids was further selected and the ClaI-XbaI fragment partially sequenced to confirm the correct junction between the mating factor $\alpha$ portion and the $Fc_\varepsilon R$-water-soluble fragment gene. This plasmid was designated as pRH 245 (see Fig. 15).

f) Preparation of the Yeast vectors

pRH 244 and pRH 245 were constructed using an E.coli vector (Bluescribe M13+) which facilitates the sequencing of the inserts. In order to express both versions of the $Fc_\varepsilon R$-water-soluble fragment gene in yeast both recombinant plasmids have to be cut with HindIII and BamHI which sets the expression cassette consisting of ADHI-promotor, MFαleader gene (in the case of pRH245), $Fc_\varepsilon R$-water-soluble fragment gene and ADHI-terminator free. These DNAs can be ligated in almost any yeast vector having the suitable restriction enzyme sites. As an example the plasmid pJDB207 (see V.D.Beggs in 'Gene cloning in yeast', Ed.R.Williamson: Genetic engineering 2, 175-203 (1982), deposited at Deutsche Sammlung von Mikroorganismen under DSM 3181)) was chosen. pJDB207 contains a 2µ origin of replication and a leu2 selection marker. This plasmid replicates extrachromosomally in yeast.

## Vector preparation

10 µg pJDB207 were doubly digested with HindIII and BamHI.
The large fragment was isolated by agarose gel electrophoresis, electroelution and precipitation. The DNA was dissolved
in 50 µl TE.

## Insert preparation

10 µg pRH 244 and pRH 245 were doubly digested with HindIII
and BamHI. The expression cassettes were also isolated using
the agarose gel electrophoresis procedure. The inserts were
dissolved in 10 µl TE.

1 µl vector and 2 µl insert were mixed and ligated in a total of 10 µl using T4-DNA ligase. E.coli JM101 was transformed with 3 µl of the ligation solution. The plasmids of some
of the resulting colonies were checked by restriction analysis for the correctness of the construction. One of each
plasmids was selected and designated as:

pJDB-244, containing the expression cassette without the MFα
leader sequence, and pJDB-245, containing the expression
cassette with the MFα leader sequence.

Both plasmids were isolated at a larger scale and used to
transform (see Beggs J.D. in Nature 275, 104 (1978)) the
yeast strain WS21-3 (α, leu2, his3, ura3, pep4).

## Beispiel 12

## Expression of the Fc$_\varepsilon$R-soluble fragment in E.coli

## Vector preparation

10 µg pRH 100 (see Example B and Himmler A., Hauptmann R.,
Adolf G. and Swetly P. in J. Interferon Res. (1986), in press) were digested with SstI. The 3'overhangs were removed by adding 5 units E.coli DNA polymerase I and dGTP. The linearised plasmid was dephosphorylated by adding 10 units CIP and by incubation at 37°C for 30 minutes. After two phenol/-chloroform extractions the DNA was further purified by agarose gel electrophoresis, electroelution and precipitation. The linearized vector was dissolved in 10 µl TE.

Insert preparation

20 µg pGEM4-Fc$_\varepsilon$R (plasmid GEM4(Promega Biotec, Madison, WI53711, USA) containing the larger HindIII-EcoRI fragment of the Fc$_\varepsilon$R-cDNA) were doubly digested with EcoRI and HindIII. The 5'overhanging ends were made blunt by addition of the Klenow fragment of DNA polymerase I and all for dXTPs. The 5'phosphate groups were removed using CIP. After agarose gel purification the fragment containing the Fc$_\varepsilon$R-soluble fragment gene was recut with Sau3A and the larger fragment isolated.

50 pMol of each oligodeoxynucleotide

  EBI-496:


5' GAACTGCAGGTGAGCTCTGGTTTCGTTTGCAACACTTGCCCGGAAAAATG      3'

  EBI-497:


3' CTTGACGTCCACTCGAGACCAAAGCAAACGTTGTGAACGGGCCTTTTTACCTAG 5'
                                                    Sau3A


restoring the reading frame starting with the second codon (Glu) of the Fc$_\varepsilon$R-soluble fragment gene and using preferred E.coli codons (Sharp P.M., Li W.-H. Nucl. Acids Res. 14, 7737-7749 (1986)) were annealed in 10 µl solution by heating to 100°C and slow cooling. The oligodeoxynucleotides and the insert DNA were ligated using T4-DNA ligase. After heat dena-
turation of the enzyme T4-polynucleotidekinase and ATP were
added in order to phosphorylate the 5'ends of the insert.
After agarose gel purification the DNA was dissolved in
10 µl TE.

1 µl linearized vector DNA and 5 µl insert DNA were combined
and ligated. Half of the material was used to transform
E.coli HB 101 (F-, hsdS20 (rb-, mb-), recA13, ara-14, proA2,
lacY1, galK2, rpsL20 (Sm-resistent), xyl-5, mtl-1, supE44,
lambda minus). The plasmids of some of the ampicillin resistent colonies were isolated and checked via restriction enzyme analysis for the correctness of the construction. One
plasmid was selected and further analysed by DNA sequencing
of the junction Trp-promotor - $Fc_\varepsilon R$-soluble fragment gene.
After this final proof the plasmid was named pRH 246 (see
Fig. 16).

Expression of the water-soluble part of $Fc_\varepsilon$-receptor can be
carried out by culturing the respective E.coli or yeast or
other organisms using standard fermentation techniques, followed by concentration and purification of the desired water-
soluble fragment using techniques described above in section
a) "isolation and purification of water-soluble part of
$Fc_\varepsilon R$".

Although in connection with the water-soluble fragment of
$Fc_\varepsilon$-receptor the construction of vectors, the transformation
of host organisms with them and the expression of the fragment have been described in detail above for the water-soluble fragment starting at amino acid 150 of the whole $Fc_\varepsilon$-re-
ceptor, it will be apparent the operations of construction,
transformation and expression can be carried out in similar
manner in order to obtain other water-soluble fragments starting at, for example, amino acids 50 to 149 of the entire
$Fc_\varepsilon$-receptor.

Figure 1 shows the $Fc_\epsilon R$ activity derived from NP-40 detergent solubilized RPMI-8866 cells and serum-free culture
supernatants (O——O) culture sup cell lysate (△——▷).

Figure 2 demonstrates the immunoaffinity purified soluble
$Fc_\epsilon R$. Immunoaffinity purified soluble $Fc_\epsilon R$ derived from
RPMI-8866 culture supernatants was analyzed by $NaDodSO_4$/PAGE
under nonreducing conditions. After electrophoresis strips
of 4 mm in width were cut, minced and eluted in lysis buffer
overnight at room temperature. The $Fc_\epsilon R$ activity was asses-
sed by an ELISA method utilizing two specific monoclonal
antibodies.

Figure 3 shows the purification of water-soluble $Fc_\epsilon R$.
Serum-free culture supernatants of RPMI-8866 cells was concentrated 200 x on Amicon YM10. Sequentially preadsorbed on
BSA-Sepharose, Transferrin-Sepharose, NMIg-Sepharose, followed by specific immunoaffinity chromatography on 3-5-Sepharose, Eluate applied to C-4 HPLC column and eluted with a
linear gradient of acetonitril O-65 % containing 0,1 % trifluoroacetic acid. Fractions containing $Fc_\epsilon R$ activity are
indicated by hatched lines.

Figure 4 shows the purified water-soluble $Fc_\epsilon$-receptor with
a molecular weight of about 25 kd. The purified soluble $Fc_\epsilon R$
was examined by SDS-PAGE analysis and a photograph of the
silver stained gel is shown.

Figure 5 shows the peptide map of the water-soluble $Fc_\epsilon R$
after lysylendopeptidase digestion and was obtained after
extensive preadsorption immunoaffinity chromatography and
HPLC.

Figure 6 shows the FACS analysis of L cell transformants.
(Left panel A), L cell transformants, L-V-8-30 were stained
with biotinated human IgG (a) or human IgE (b) and developed with FITC-avidin. (Right panel B), The same cells were stained with phycocyanin conjugated control, irrelevant murine IgG$_1$ antibodiy (c), or phycocyanin conjugated anti-Fc$_\varepsilon$R (3-5) (d). Unstained cells showed the same pattern as those stained with control antibodies (a and c). Y and X axes represent relative cell numbers and log fluorescence intensities respectively.

Figure 7 shows the strategy for cDNA cloning.

Figure 8 shows the EcoRI-insert in the plasmid pDE2, named as pDE2-Fc$_\varepsilon$R-1 vector.

Figure 8' shows the expression of Fc$_\varepsilon$R cDNA in transfected Cos-7 cells. The transfected Cos-7 cells were stained with phycocyanin-conjugated anti-Fc$_\varepsilon$R antibody (3-5) and biotinated IgE, developed with FITC-avidin and analyzed by a dual laser FACS; a) cells transfected with pDE2 containing human IFN-ß cDNA; b) cells transfected with pDE-2-Fc$_\varepsilon$R-1. Contour plots represent the correlated expression of two surface determinants by showing peak lines enclosing equal percentage of cells with the two parameter distribution. X and Y axes represent the green and red log fluorescence intensities respectively.

Figure 9 shows the EcoRI-insert in the plasmid pGEM-4.

Figure 9' shows the expression of a Fc$_\varepsilon$R cDNA in Xenopus oocyctes. Oocytes injected with mRNA transcript of pFc$_\varepsilon$R-1, control mRNA or with mRNA from 8866 cells were incubated for 2 days and lysed, the levels of Fc$_\varepsilon$R in lysates were measured by ELISA.

Table 1:         Purification of Fc R from RPMI-8866 cells

| Material | Total Protein (µg) | Total Activity (units*) | Specific Activity units/µg | Percent Recovery | Purifi-cation |
|---|---|---|---|---|---|
| Cell culture Supernatant | 180.000 | 78.800 | 0.44 | 100 | 1 |
| Concentrated Supernatant (190X) | 172.000 | 75.000 | 0.44 | 95.2 | 1 |
| NMIg Effluent | 132.000 | 55.000 | 0.42 | 70 | 1 |
| 3-5-Seph. Eluate | 441 | 36.800 | 83.4 | 47 | 190 |
| HPLC | 16 | 26.000 | 1.630 | 33 | 3.710 |
| Crude Cell lysate | 117.000 | 6.160 | 0.05 | 100 | 1 |
| NMIg Effluent | 99.000 | 2.475 | 0.02 | 40.2 | 0.47 |
| 3-5-Seph. Eluate | 306 | 3.795 | 12.4 | 61.6 | 236 |
| HPLC-purified | – | 649 | 649 | 10.5 | 12.400 |

* 1 unit is the activity of $1 \times 10^5$ cell equivalent of 190X concentrated culture supernatant.

Table 2

| Material | 3-5-Sepharose | | NMIg-Sepharose | | IgE-Sepharose | |
|---|---|---|---|---|---|---|
| | Eluate | Effluent | Eluate | Effluent | Eluate | Effluent |
| HPLC purfied | 2,470 | 4.5 | 85.5 | 1,170 | | |

## Table 3

```
        CTCCT GCTTAAACCT CTGTCTCTGA CGGTCCCTGC          35
        GAGGA CGAATTTGGA GACAGAGACT GCCAGGGACG

CAATCGCTCT GGTCGACCCC AACACACTAG GAGGACAGAC ACAGGCTCCA    85
GTTAGCGAGA CCAGCTGGGG TTGTGTGATC CTCCTGTCTG TGTCCGAGGT

AACTCCACTA AGTGACCAGA GCTGTGATTG TGCCCGCTGA GTGGACTGCG   135
TTGAGGTGAT TCACTGGTCT CGACACTAAC ACGGGCGACT CACCTGACGC

TTGTCAGGGA GTGAGTGCTC CATCATCGGG AGAATCCAAG CAGGACCGCC   185
AACAGTCCCT CACTCACGAG GTAGTAGCCC TCTTAGGTTC GTCCTGGCGG
```

```
                      5                  10                 15
Met Glu Glu Gly Gln Tyr Ser Glu Ile Glu Glu Leu Pro Arg Arg
ATG GAG GAA GGT CAA TAT TCA GAG ATC GAG GAG CTT CCC AGG AGG   230
TAC CTC CTT CCA GTT ATA AGT CTC TAG CTC CTC GAA GGG TCC TCC


                     20                  25                 30
Arg Cys Cys Arg Arg Gly Thr Gln Ile Val Leu Leu Gly Leu Val
CGG TGT TGC AGG CGT GGG ACT CAG ATC GTG CTG CTG GGG CTG GTG   275
GCC ACA ACG TCC GCA CCC TGA GTC TAG CAC GAC GAC CCC GAC CAC


                     35                  40                 45
Thr Ala Ala Leu Trp Ala Gly Leu Leu Thr Leu Leu Leu Leu Trp
ACC GCC GCT CTG TGG GCT GGG CTG CTG ACT CTG CTT CTC CTG TGG   320
TGG CGG CGA GAC ACC CGA CCC GAC GAC TGA GAC GAA GAG GAC ACC


                     50                  55                 60
His Trp Asp Thr Thr Gln Ser Leu Lys Gln Leu Glu Glu Arg Ala
CAC TGG GAC ACC ACA CAG AGT CTA AAA CAG CTG GAA GAG AGG GCT   365
GTG ACC CTG TGG TGT GTC TCA GAT TTT GTC GAC CTT CTC TCC CGA


                     65                  70                 75
Ala Arg Asn Val Ser Gln Val Ser Lys Asn Leu Glu Ser His His
GCC CGG AAC GTC TCT CAA GTT TCC AAG AAC TTG GAA AGC CAC CAC   410
CGG GCC TTG CAG AGA GTT CAA AGG TTC TTG AAC CTT TCG GTG GTG


                     80                  85                 90
Gly Asp Gln Met Ala Gln Lys Ser Gln Ser Thr Gln Ile Ser Gln
GGT GAC CAG ATG GCG CAG AAA TCC CAG TCC ACG CAG ATT TCA CAG   455
CCA CTG GTC TAC CGC GTC TTT AGG GTC AGG TGC GTC TAA AGT GTC


                     95                 100                105
Glu Leu Glu Glu Leu Arg Ala Glu Gln Gln Arg Leu Lys Ser Gln
GAA CTG GAG GAA CTT CGA GCT GAA CAG CAG AGA TTG AAA TCT CAG   500
CTT GAC CTC CTT GAA GCT CGA CTT GTC GTC TCT AAC TTT AGA GTC


                    110                 115                120
Asp Leu Glu Leu Ser Trp Asn Leu Asn Gly Leu Gln Ala Asp Leu
GAC TTG GAG CTG TCC TGG AAC CTG AAC GGG CTT CAA GCA GAT CTG   545
CTG AAC CTC GAC AGG ACC TTG GAC TTG CCC GAA GTT CGT CTA GAC
```

```
                125                  130                  135
Ser Ser Phe Lys Ser Gln Glu Leu Asn Glu Arg Asn Glu Ala Ser
AGC AGC TTC AAG TCC CAG GAA TTG AAC GAG AGG AAC GAA GCT TCA  590
TCG TCG AAG TTC AGG GTC CTT AAC TTG CTC TCC TTG CTT CGA AGT


                140                  145                  150
Asp Leu Leu Glu Arg Leu Arg Glu Glu Val Thr Lys Leu Arg Met
GAT TTG CTG GAA AGA CTC CGG GAG GAG GTG ACA AAG CTA AGG ATG  635
CTA AAC GAC CTT TCT GAG GCC CTC CTC CAC TGT TTC GAT TCC TAC


                155                  160                  165
Glu Leu Gln Val Ser Ser Gly Phe Val Cys Asn Thr Cys Pro Glu
GAG TTG CAG GTG TCC AGC GGC TTT GTG TGC AAC ACG TGC CCT GAA  680
CTC AAC GTC CAC AGG TCG CCG AAA CAC ACG TTG TGC ACG GGA CTT


                170                  175                  180
Lys Trp Ile Asn Phe Gln Arg Lys Cys Tyr Tyr Phe Gly Lys Gly
AAG TGG ATC AAT TTC CAA CGG AAG TGC TAC TAC TTC GGC AAG GGC  725
TTC ACC TAG TTA AAG GTT GCC TTC ACG ATG ATG AAG CCG TTC CCG


                185                  190                  195
Thr Lys Gln Trp Val His Ala Arg Tyr Ala Cys Asp Asp Met Glu
ACC AAG CAG TGG GTC CAC GCC CGG TAT GCC TGT GAC GAC ATG GAA  770
TGG TTC GTC ACC CAG GTG CGG GCC ATA CGG ACA CTG CTG TAC CTT


                200                  205                  210
Gly Gln Leu Val Ser Ile His Ser Pro Glu Glu Gln Asp Phe Leu
GGG CAG CTG GTC AGC ATC CAC AGC CCG GAG GAG CAG GAC TTC CTG  815
CCC GTC GAC CAG TCG TAG GTG TCG GGC CTC CTC GTC CTG AAG GAC


                215                  220                  225
Thr Lys His Ala Ser His Thr Gly Ser Trp Ile Gly Leu Arg Asn
ACC AAG CAT GCC AGC CAC ACC GGC TCC TGG ATT GGC CTT CGG AAC  860
TGG TTC GTA CGG TCG GTG TGG CCG AGG ACC TAA CCG GAA GCC TTG


                230                  235                  240
Leu Asp Leu Lys Gly Glu Phe Ile Trp Val Asp Gly Ser His Val
TTG GAC CTG AAG GGA GAG TTT ATC TGG GTG GAT GGG AGC CAT GTG  905
AAC CTG GAC TTC CCT CTC AAA TAG ACC CAC CTA CCC TCG GTA CAC


                245                  250                  255
Asp Tyr Ser Asn Trp Ala Pro Gly Glu Pro Thr Ser Arg Ser Gln
GAC TAC AGC AAC TGG GCT CCA GGG GAG CCC ACC AGC CGG AGC CAG  950
CTG ATG TCG TTG ACC CGA GGT CCC CTC GGG TGG TCG GCC TCG GTC


                260                  265                  270
Gly Glu Asp Cys Val Met Met Arg Gly Ser Gly Arg Trp Asn Asp
GGC GAG GAC TGC GTG ATG ATG CGG GGC TCC GGT CGC TGG AAC GAC  995
CCG CTC CTG ACG CAC TAC TAC GCC CCG AGG CCA GCG ACC TTG CTG


                275                  280                  285
Ala Phe Cys Asp Arg Lys Leu Gly Ala Trp Val Cys Asp Arg Leu
GCC TTC TGC GAC CGT AAG CTG GGC GCC TGG GTG TGC GAC CGG CTG 1040
CGG AAG ACG CTG GCA TTC GAC CCG CGG ACC CAC ACG CTG GCC GAC
```

```
                        290               295                 300
Ala Thr Cys Thr Pro Pro Ala Ser Glu Gly Ser Ala Glu Ser Met
GCC ACA TGC ACG CCG CCA GCC AGC GAA GGT TCC GCG GAG TCC ATG 1085
CGG TGT ACG TGC GGC GGT CGG TCG CTT CCA AGG CGC CTC AGG TAC

                        305               310                 315
Gly Pro Asp Ser Arg Pro Asp Pro Asp Gly Arg Leu Pro Thr Pro
GGA CCT GAT TCA AGA CCA GAC CCT GAC GGC CGC CTG CCC ACC CCC 1130
CCT GGA CTA AGT TCT GGT CTG GGA CTG CCG GCG GAC GGG TGG GGG

                                    *
Ser Ala Pro Leu His Ser
TCT GCC CCT CTC CAC TCT TGA GCATGGATA CAGCCAGGCC CAGAGCAAGA 1180
AGA CGG GGA GAG GTG AGA ACT CGTACCTAT GTCGGTCCGG GTCTCGTTCT

CCCTGAAGAC CCCCAACCAC GGCCTAAAAG CCTCTTTGTG GCTGAAAGGT      1230
GGGACTTCTG GGGGTTGGTG CCGGATTTTC GGAGAAACAC CGACTTTCCA

CCCTGTGACA TTTTCTGCCA CCCAAACGGA GGCAGCTGAC ACATCTCCCG      1280
GGGACACTGT AAAAGACGGT GGGTTTGCCT CCGTCGACTG TGTAGAGGGC

CTCCTCTATG GCCCCTGCCT TCCCAGGAGT ACACCCCAAC AGCACCCTCT      1330
GAGGAGATAC CGGGGACGGA AGGGTCCTCA TGTGGGGTTG TCGTGGGAGA

CCAGATGGGA GTGCCCCCAA CAGCACCCTC TCCAGATGAG AGTACACCCC      1380
GGTCTACCCT CACGGGGGTT GTCGTGGGAG AGGTCTACTC TCATGTGGGG

AACAGCACCC TCTCCAGATG CAGCCCCATC TCCTCAGCAC CCCAGGACCT      1430
TTGTCGTGGG AGAGGTCTAC GTCGGGGTAG AGGAGTCGTG GGGTCCTGGA

GAGTATCCCC AGCTCAGGTG GTGAGTCCTC CTGTCCAGCC TGCATCAATA      1480
CTCATAGGGG TCGAGTCCAC CACTCAGGAG GACAGGTCGG ACGTAGTTAT

                                                            1504
AAATGGGGCA GTGATGGCCT CCCA
TTTACCCCGT CACTACCGGA GGGT
```

We claim:

1. Human low affinity Fc$_\varepsilon$-receptor with a N-terminal cytoplasmic domain, a C-terminal extracellular domain and a molecular weight of about 46 kd and the glycosylated derivate thereof.

2. Human low affinity Fc$_\varepsilon$-receptor as claimed in claim 1 containing the following partial amino acid sequences:

Met-Glu-Leu-Gln-Val-Ser-Ser-Gly-Phe-Val-,

Gly-Glu-Phe-Ile-Trp-Val-Asp-Gly-Ser-His-Val-Asp-Tyr-Ser-Asn-Trp-Ala-Pro-Gly-Glu-Pro-Thr-,

Lys-His-Ala-Ser-His-Thr-Gly-Ser-Trp-Ile-Gly-Leu-Arg-Asn-Leu-Asp-Leu-Lys- and

Lys-Trp-Ile-Asn-Phe-Gln-.

3. Human low affinity Fc$_\varepsilon$-receptor as claimed in claim 1 containing the partial amino acid sequence of the formula

Lys-Trp-Ile-Asn-Phe-Gln-.

4. Human low affinity Fc$_\varepsilon$-receptor as claimed in any of the claims 1 to 3 having the formula

```
Met Glu Glu Gly Gln Tyr Ser Glu Ile Glu Glu Leu Pro Arg Arg
Arg Cys Cys Arg Arg Gly Thr Gln Ile Val Leu Leu Gly Leu Val
Thr Ala Ala Leu Trp Ala Gly Leu Leu Thr Leu Leu Leu Leu Trp
His Trp Asp Thr Thr Gln Ser Leu Lys Gln Leu Glu Glu Arg Ala
Ala Arg Asn Val Ser Gln Val Ser Lys Asn Leu Glu Ser His His
Gly Asp Gln Met Ala Gln Lys Ser Gln Ser Thr Gln Ile Ser Gln
Glu Leu Glu Glu Leu Arg Ala Glu Gln Gln Arg Leu Lys Ser Gln
Asp Leu Glu Leu Ser Trp Asn Leu Asn Gly Leu Gln Ala Asp Leu
Ser Ser Phe Lys Ser Gln Glu Leu Asn Glu Arg Asn Glu Ala Ser
Asp Leu Leu Glu Arg Leu Arg Glu Glu Val Thr Lys Leu Arg Met
```

```
Glu Leu Gln Val Ser Ser Gly Phe Val Cys Asn Thr Cys Pro Glu
Lys Trp Ile Asn Phe Gln Arg Lys Cys Tyr Tyr Phe Gly Lys Gly
Thr Lys Gln Trp Val His Ala Arg Tyr Ala Cys Asp Asp Met Glu
Gly Gln Leu Val Ser Ile His Ser Pro Glu Glu Gln Asp Phe Leu
Thr Lys His Ala Ser His Thr Gly Ser Trp Ile Gly Leu Arg Asn
Leu Asp Leu Lys Gly Glu Phe Ile Trp Val Asp Gly Ser His Val
Asp Tyr Ser Asn Trp Ala Pro Gly Glu Pro Thr Ser Arg Ser Gln
Gly Glu Asp Cys Val Met Met Arg Gly Ser Gly Arg Trp Asn Asp
Ala Phe Cys Asp Arg Lys Leu Gly Ala Trp Val Cys Asp Arg Leu
Ala Thr Cys Thr Pro Pro Ala Ser Glu Gly Ser Ala Glu Ser Met
Gly Pro Asp Ser Arg Pro Asp Pro Asp Gly Arg Leu Pro Thr Pro
Ser Ala Pro Leu His Ser
```

5. Water-soluble part of human low affinity Fc$_\varepsilon$-receptor.

6. Water-soluble Fc$_\varepsilon$-receptor part as claimed in claim 5 starting from an amino acid corresponding to 50 to about 150 of the amino acid sequence as claimed in claim 4 and the glycosylated derivate thereof.

7. Water-soluble Fc$_\varepsilon$-receptor part as claimed in claim 5 or 6 containing the amino acid sequence of formula

```
                                                            Met
Glu Leu Gln Val Ser Ser Gly Phe Val Cys Asn Thr Cys Pro Glu
Lys Trp Ile Asn Phe Gln Arg Lys Cys Tyr Tyr Phe Gly Lys Gly
Thr Lys Gln Trp Val His Ala Arg Tyr Ala Cys Asp Asp Met Glu
Gly Gln Leu Val Ser Ile His Ser Pro Glu Glu Gln Asp Phe Leu
Thr Lys His Ala Ser His Thr Gly Ser Trp Ile Gly Leu Arg Asn
Leu Asp Leu Lys Gly Glu Phe Ile Trp Val Asp Gly Ser His Val
Asp Tyr Ser Asn Trp Ala Pro Gly Glu Pro Thr Ser Arg Ser Gln
Gly Glu Asp Cys Val Met Met Arg Gly Ser Gly Arg Trp Asn Asp
Ala Phe Cys Asp Arg Lys Leu Gly Ala Trp Val Cys Asp Arg Leu
Ala Thr Cys Thr Pro Pro Ala Ser Glu Gly Ser Ala Glu Ser Met
Gly Pro Asp Ser Arg Pro Asp Pro Asp Gly Arg Leu Pro Thr Pro
Ser Ala Pro Leu His Ser
```

8. Water-soluble Fc$_\varepsilon$-receptor part as claimed in any of the claims 5 to 7 having the formula as claimed in claim 7.

9. Recombinant human low affinity Fc$_\varepsilon$-receptor or a water-soluble part thereof as claimed in any of the claims 1 to 8, essentially free of other proteins of human origin.

10. Recombinant human low affinity Fc$_\epsilon$-receptor as claimed in claim 9 unaccompanied by associated native glycosylation.

11. Recombinant human low affinity Fc$_\epsilon$-receptor as claimed in the claims 9 and 10 produced by expression of the DNA-sequence of formula

```
ATG GAG GAA GGT CAA TAT TCA GAG ATC GAG GAG CTT CCC AGG AGG
TAC CTC CTT CCA GTT ATA AGT CTC TAG CTC CTC GAA GGG TCC TCC

CGG TGT TGC AGG CGT GGG ACT CAG ATC GTG CTG CTG GGG CTG GTG
GCC ACA ACG TCC GCA CCC TGA GTC TAG CAC GAC GAC CCC GAC CAC

ACC GCC GCT CTG TGG GCT GGG CTG CTG ACT CTG CTT CTC CTG TGG
TGG CGG CGA GAC ACC CGA CCC GAC GAC TGA GAC GAA GAG GAC ACC

CAC TGG GAC ACC ACA CAG AGT CTA AAA CAG CTG GAA GAG AGG GCT
GTG ACC CTG TGG TGT GTC TCA GAT TTT GTC GAC CTT CTC TCC CGA

GCC CGG AAC GTC TCT CAA GTT TCC AAG AAC TTG GAA AGC CAC CAC
CGG GCC TTG CAG AGA GTT CAA AGG TTC TTG AAC CTT TCG GTG GTG

GGT GAC CAG ATG GCG CAG AAA TCC CAG TCC ACG CAG ATT TCA CAG
CCA CTG GTC TAC CGC GTC TTT AGG GTC AGG TGC GTC TAA AGT GTC

GAA CTG GAG GAA CTT CGA GCT GAA CAG CAG AGA TTG AAA TCT CAG
CTT GAC CTC CTT GAA GCT CGA CTT GTC GTC TCT AAC TTT AGA GTC

GAC TTG GAG CTG TCC TGG AAC CTG AAC GGG CTT CAA GCA GAT CTG
CTG AAC CTC GAC AGG ACC TTG GAC TTG CCC GAA GTT CGT CTA GAC

AGC AGC TTC AAG TCC CAG GAA TTG AAC GAG AGG AAC GAA GCT TCA
TCG TCG AAG TTC AGG GTC CTT AAC TTG CTC TCC TTG CTT CGA AGT

GAT TTG CTG GAA AGA CTC CGG GAG GAG GTG ACA AAG CTA AGG ATG
CTA AAC GAC CTT TCT GAG GCC CTC CTC CAC TGT TTC GAT TCC TAC

GAG TTG CAG GTG TCC AGC GGC TTT GTG TGC AAC ACG TGC CCT GAA
CTC AAC GTC CAC AGG TCG CCG AAA CAC ACG TTG TGC ACG GGA CTT

AAG TGG ATC AAT TTC CAA CGG AAG TGC TAC TAC TTC GGC AAG GGC
TTC ACC TAG TTA AAG GTT GCC TTC ACG ATG ATG AAG CCG TTC CCG

ACC AAG CAG TGG GTC CAC GCC CGG TAT GCC TGT GAC GAC ATG GAA
TGG TTC GTC ACC CAG GTG CGG GCC ATA CGG ACA CTG CTG TAC CTT

GGG CAG CTG GTC AGC ATC CAC AGC CCG GAG GAG CAG GAC TTC CTG
CCC GTC GAC CAG TCG TAG GTG TCG GGC CTC CTC GTC CTG AAG GAC

ACC AAG CAT GCC AGC CAC ACC GGC TCC TGG ATT GGC CTT CGG AAC
TGG TTC GTA CGG TCG GTG TGG CCG AGG ACC TAA CCG GAA GCC TTG
```

```
TTG GAC CTG AAG GGA GAG TTT ATC TGG GTG GAT GGG AGC CAT GTG
AAC CTG GAC TTC CCT CTC AAA TAG ACC CAC CTA CCC TCG GTA CAC

GAC TAC AGC AAC TGG GCT CCA GGG GAG CCC ACC AGC CGG AGC CAG
CTG ATG TCG TTG ACC CGA GGT CCC CTC GGG TGG TCG GCC TCG GTC

GGC GAG GAC TGC GTG ATG ATG CGG GGC TCC GGT CGC TGG AAC GAC
CCG CTC CTG ACG CAC TAC TAC GCC CCG AGG CCA GCG ACC TTG CTG

GCC TTC TGC GAC CGT AAG CTG GGC GCC TGG GTG TGC GAC CGG CTG
CGG AAG ACG CTG GCA TTC GAC CCG CGG ACC CAC ACG CTG GCC GAC

GCC ACA TGC ACG CCG CCA GCC AGC GAA GGT TCC GCG GAG TCC ATG
CGG TGT ACG TGC GGC GGT CGG TCG CTT CCA AGG CGC CTC AGG TAC

GGA CCT GAT TCA AGA CCA GAC CCT GAC GGC CGC CTG CCC ACC CCC
CCT GGA CTA AGT TCT GGT CTG GGA CTG CCG GCG GAC GGG TGG GGG

TCT GCC CCT CTC CAC TCT TGA
AGA CGG GGA GAG GTG AGA ACT
```

or of a degenerative derivate thereof.


12. Recombinant water-soluble part of human lower affinity
Fc$_\epsilon$-receptor as claimed in claims 9 and 10 produced by expression of a DNA-sequence starting with the codons from
about 50 to about 150 of the DNA-sequence as claimed in
claim 11.


13. Recombinant water-soluble part of human lower affinity
Fc$_\epsilon$-receptor as claimed in claims 9 and 10 produced by expression of a DNA-sequence containing at least the sequence
of formula

```
                                                        ATG
                                                        TAC

GAG TTG CAG GTG TCC AGC GGC TTT GTG TGC AAC ACG TGC CCT GAA
CTC AAC GTC CAC AGG TCG CCG AAA CAC ACG TTG TGC ACG GGA CTT

AAG TGG ATC AAT TTC CAA CGG AAG TGC TAC TAC TTC GGC AAG GGC
TTC ACC TAG TTA AAG GTT GCC TTC ACG ATG ATG AAG CCG TTC CCG

ACC AAG CAG TGG GTC CAC GCC CGG TAT GCC TGT GAC GAC ATG GAA
TGG TTC GTC ACC CAG GTG CGG GCC ATA CGG ACA CTG CTG TAC CTT

GGG CAG CTG GTC AGC ATC CAC AGC CCG GAG GAG CAG GAC TTC CTG
CCC GTC GAC CAG TCG TAG GTG TCG GGC CTC CTC GTC CTG AAG GAC
```

```
ACC AAG CAT GCC AGC CAC ACC GGC TCC TGG ATT GGC CTT CGG AAC
TGG TTC GTA CGG TCG GTG TGG CCG AGG ACC TAA CCG GAA GCC TTG

TTG GAC CTG AAG GGA GAG TTT ATC TGG GTG GAT GGG AGC CAT GTG
AAC CTG GAC TTC CCT CTC AAA TAG ACC CAC CTA CCC TCG GTA CAC

GAC TAC AGC AAC TGG GCT CCA GGG GAG CCC ACC AGC CGG AGC CAG
CTG ATG TCG TTG ACC CGA GGT CCC CTC GGG TGG TCG GCC TCG GTC

GGC GAG GAC TGC GTG ATG ATG CGG GGC TCC GGT CGC TGG AAC GAC
CCG CTC CTG ACG CAC TAC TAC GCC CCG AGG CCA GCG ACC TTG CTG

GCC TTC TGC GAC CGT AAG CTG GGC GCC TGG GTG TGC GAC CGG CTG
CGG AAG ACG CTG GCA TTC GAC CCG CGG ACC CAC ACG CTG GCC GAC

GCC ACA TGC ACG CCG CCA GCC AGC GAA GGT TCC GCG GAG TCC ATG
CGG TGT ACG TGC GGC GGT CGG TCG CTT CCA AGG CGC CTC AGG TAC

GGA CCT GAT TCA AGA CCA GAC CCT GAC GGC CGC CTG CCC ACC CCC
CCT GGA CTA AGT TCT GGT CTG GGA CTG CCG GCG GAC GGG TGG GGG

TCT GCC CCT CTC CAC TCT TGA
AGA CGG GGA GAG GTG AGA ACT
```

or of a degenerative derivate thereof.

14. Recombinant water-soluble part of human lower affinity Fc$_\epsilon$-receptor as claimed in claims 9 and 10 produced by expression of the DNA-sequence as claimed in claim 13.

15. A recombinant DNA molecule which contains the genetic information as claimed in any of the claims 1 to 14 or a degenerative derivate thereof.

16. A recominant DNA molecule as claimed in claim 15 which contains the genetic information as claimed in claim 11 or 14.

17. A recombinant DNA molecule as claimed in claim 15 or 16 containing additionally the replicon and control sequences necessary for expression.

18. A recombinant DNA molecule as claimed in claim 17 containing the replicon and control sequences for expression in prokaryotes, eukaryotes or in mammalian cells.

0258492

19. A recombinant DNA molecule as claimed in claim 17 or 18 containing the replicon and control sequences suitable for expression in E.coli or in yeast.

20. A recombinant DNA molecule as claimed in claim 19 wherein as replicon and control sequences those of plasmid pER103 are used.

21. A recombinant DNA molecule as claimed in claim 19 containing as replicon the 2μ origin and as control sequences the ADHI-promotor and the ADHI-terminator.

22. A recombinant DNA molecule as claimed in claim 21 containing additionally the mating factor α leader sequence (MFα).

23. A vector containing a recombinant DNA-molecule as claimed in any of the claims 15 to 22.

24. A vector containing a recombinant DNA-molecule as claimed in any of the claims 16 to 22.

25. A vector as claimed in claim 23 or 24 wherein the vector is a plasmid vector.

26. A plasmid as claimed in claim 25 containing the coding sequence as claimed in any of the claims 11 to 14.

27. The plasmid LE392 as claimed in claim 26 deposited in E.coli HB101 under FERM BP-1116 containing the DNA-sequence as claimed in claim 11 within the plasmid pGEM$^{TM}$-4 as EcoRI-insert.

28. The plasmid pDE2-Fc$_{\epsilon}$R-1 as claimed in claim 26 containing the DNA-sequence as claimed in claim 11 within the plasmid pDE2 as EcoRI-insert.

29. The plasmid pRH246 as claimed in claim 26 containing the DNA sequences as claimed in claim 14 and 20 within the plasmid pBR322 as EcoRI-insert having the restriction map as shown in Fig. 16.

30. The plasmid pRH244 as claimed in claim 26 containing the DNA-sequences as claimed in claim 14 and 21 within the plasmid Bluescribe M13+ as BamHI/HindIII-insert having the restriction map as shown in Fig. 14.

31. The plasmid pRH245 as claimed in claim 26 containing the DNA sequences as claimed in claim 14 and claim 21 within the plasmid Bluescribe M13+ as BamHI/HindIII-insert having the restriction map as shown in Fig. 15.

32. An yeast vector as claimed in claim 25 containing the coding plasmid DNA as claimed in claim 30 or 31.

33. A yeast vector as claimed in claim 32 designated as pJDB-244 containing the coding DNA as claimed in claim 30 as BamHI/HindIII-insert within the plasmid pJDB207.

34. A yeast vector as claimed in claim 32 designated as pJDB245 containing the coding DNA as claimed in claim 31 as BamHI/HindIII-insert within the plasmid pJDB207.

35. A host organism transformed by a vector as claimed in any of the claims 23 to 34.

36. An oligonucleotide encoding for a partial amino acid sequence as claimed in claim 2 or 3.

37. The oligonucleotide as claimed in claim 36 showing the formula

$$3'-TT^T_C \ ACC \ TAG^T_A \ TT^A_G \ AA^A_G \ GT \ -5'$$

, which encodes for a part of the amino acid sequence as
claimed in claim 3.

38. A process for preparing a polypeptide as claimed in any
one of claims 1 to 8 which comprises transforming a suitable
host organism with an expression vector containing a coding
sequence as claimed in any of the claims 9 to 14 for the desired polypeptide at an appropriate site for expression and
isolating the desired polypeptide from the resulting trans-
formants.

39. A process as claimed in claim 37 wherein an expression
vector used as claimed in any of the claims 24 to 34.

40. Process for the preparation of human low affinity $Fc_{\epsilon}$-re-
ceptor as claimed in any of the claims 5 to 8 or a water-
soluble part thereof which comprises

culturing B lymphoblastoid cells and separating said $Fc_{\epsilon}R$
from the supernatant or from the lysed cells by sequential
immunoaffinity purification.

41. Process as claimed in claim 40, wherein RPMI-8866 cells
are used as lymphoblastoid cells.

42. Process as claimed in claims 40 or 41, wherein a solu-
tion containing $Fc_{\epsilon}R$ is sequentially absorbed on BSA-Sepharose, human transferrin Sepharose and normal mouse Ig-Sepharose, the effluent is applied to an anti-$Fc_{\epsilon}R$-affinity co-
lumn and the eluent is further purified by means of HPLC.

43. Process as claimed in claim 42, wherein the immunoaffinity column is prepared by coupling a corresponding antibody
to Sepharose.

44. Process for the preparation of partial amino acid sequences as claimed in claims 2 or 3 wherein $Fc_{\varepsilon}$-receptor as prepared according to claim 42 is subjected to trypsin or lysylendopeptidase treatment and subsequently separated by means of HPLC.

45. Process as claimed in claim 44, wherein the partial amino acid sequence as claimed in claim 3 is isolated.

46. Process for the preparation of the DNA-sequence as claimed in claim 36 or 37 wherein the oligonucleotide is synthesized by addition of the corresponding nucleotides by mean of an oligo synthesizer.

47. A process for identifying expression vehicles containing genes coding for $Fc_{\varepsilon}R$ as claimed in claims 1 to 8, comprising the steps of:

synthesizing cDNA from an RNA matrix derived from lymphoblastoid cells producing $Fc_{\varepsilon}R$ mRNA,

incorporating said synthesized cDNA in expression vehicles to form an expression vehicle bank,

hybridizing said incorporated cDNA to identify those expression vehicles which contain a gene coding for $Fc_{\varepsilon}R$, with two labelled probes comprising cDNA specific to $Fc_{\varepsilon}R^{+}L$ cell and an oligonucleotide common to gene of $Fc_{\varepsilon}R$.

48. Process as claimed in claim 47, wherein said probe I is synthesized from a matrix of RNA, which was isolated from $Fc_{\varepsilon}R^{+}L$ cells.

49. Process as claimed in claim 47, wherein said probe II is synthesized from a matrix of RNA containing $Fc_{\varepsilon}R$ sequences and said oligonucleotide as claimed in claim 35 or 36 is a primer.

50. Process as claimed in claim 47, wherein said lymphoblastoid cell are cells of line RPMI-8866.

51. Process as claimed in claim 47, wherein said $Fc_\epsilon R^+L$ cells are obtained by co-transfection of $Ltk^-$ cells with Herpes simplex virus derived tk gene and high molecules genomic DNA from RPMI-8866 cells.

52. Process for preparing a host organism as claimd in claim 35, wherein a vector as claimed in claims 23 to 34 is transformed with a suitable host.

53. Process for preparing a vector as claimed in claims 23 to 34, wherein a DNA-sequence as claimed in claims 15 to 22 is inserted in a suitable vector.

54. Pharmaceutical compositions containing a polypeptide as claimed in claim 1 to 14.

55. Pharmaceutical compositions as claimed in claim 54 suitable for treatment of local or systhemic IgE-allergic reactions.

56. Preparation of a pharmaceutical composition as claimed in claims 54 or 55 wherein an effective amount of a polypeptide as claimed in any of the claims 1 to 14 is incorporated in one or more excipients.

57. Use of a polypeptide as claimed in any of the claims 1 to 14 for the preparation of a pharmaceutical composition.

0258492

1/14

Figure 1

FcER activity in cell lysate and sup.
of RPMI8866 cells

Figure 2

Figure 3

0258492

3/14

4/14

Fig. 4

Fig. 5

Fig. 6

A

B

Relative number of cells

Fluorescence intensity

L-V-8-30 (λ)

L-V-8-30 (ε)

(a,d) control
(b,e) IgE
(c,f) anti-FcεR (8-30)

Fig. 7  Strategy for cDNA cloning

Probe I
Cellular DNA from RPMI 8866
    ↓ Transfection
Ltk⁻ cells
    ↓ FACS sorting
FcεR'
transformant

L cells

    ↓        ↓
cDNA  —  mRNA
         ↓

| Transformant specific cDNA |

Probe II
Culture supernatant
from RPMI 8866
    ↓ Purification
Purifed soluble FcεR
    ↓
Amino acid sequences
    ↓

| Synthetic oligonucleotides (17mer) |

RPMI 8866 ——→ mRNA ——→ | cDNA library in λgt10 |     Colony hybridization
cells
                              ↓
                         | cDNA clone for FcεR |

Fig. 8

FcεR cDNA

EcoRI          EcoRI

SV40 early     EcoRI   EcoRI   SV40 early

pDE-2

Anti-FcεR (3-5)

pDE2-FcεR cDNA
transfected COS-7

Anti-FcεR (3-5)

pDE2 transfected
.COS-7

Biotinated IgE

Fig. 8'

0258492

Fig. 9'

O.D. 1.0

0.5

FcεR cDNA transcript
injected oocytes

Control mRNA
injected oocytes

$3.3 \times 10^5$   $1 \times 10^5$   $3.3 \times 10^4$

FcεR from 8866 cells

Fig. 9

FcεR cDNA

EcoRI          EcoRI

EcoRI

SP6 promotor   MCS   T7 promotor

pGEM™-4

Fig.10

Fig.11

Fig.13

0258492

Fig. 12

```
                                              MF1
                                              TCGAGCCTCATATCA
                                                 CGGAGTATAGT
                                                   XhoI


                                              MF3
Met Arg Phe Pro Ser Ile Phe Thr Ala Val Leu Phe Ala Ala Ser
ATG AGA TTC CCA TCT ATT TTC ACT GCT GTT TTG TTC GCT GCT TCC
TAC TCT AAG GGT AGA TAA AAG TGA CGA CAA AAC AAG CGA CGA AGG
                                                      MF2


Ser Ala Leu Ala Ala Pro Val Asn Thr Thr Thr Glu Asp Glu Thr
TCC GCT TTG GCT GCT CCA GTC AAC ACT ACT ACT GAA GAC GAA ACT
AGG CGA AAC CGA CGA GGT CAG TTG TGA TGA TGA CTT CTG CTT TGA


                        MF5
Ala Gln Ile Pro Ala Glu Ala Val Ile Gly Tyr Ser Asp Leu Glu
GCT CAA ATT CCA GCT GAA GCT GTC ATC GGT TAC TCT GAC TTG GAA
CGA GTT TAA GGT CGA CTT CGA CAG TAG CCA ATG AGA CTG AAC CTT
                            MF4


                    MF7
Gly Asp Phe Asp Val Ala Val Leu Pro Phe Ser Asn Ser Thr Asn
GGT GAC TTC GAC GTT GCT GTT TTG CCA TTC TCC AAC TCC ACT AAC
CCA CTG AAG CTG CAA CGA CAA AAC GGT AAG AGG TTG AGG TGA TTG
                            MF6


                                                          MF9
Asn Gly Leu Leu Phe Ile Asn Thr Thr Ile Ala Ser Ile Ala Ala
AAC GGT TTG TTG TTC ATT AAC ACT ACT ATT GCA TCG ATT GCT GCT
TTG CCA AAC AAC AAG TAA TTG TGA TGA TAA CGT AGC TAA CGA CGA
                                                  ClaI


Lys Glu Glu Gly Val Ser Leu Asp Lys Arg
AAG GAA GAA GGT GTT TCT TTG GAC AAG AGG CCTCTGCAGGAATTCT
TTC CTT CTT CCA CAA AGA AAC CTG TTC TCC GGAGACGTCCTTAAGAGATC
                                          StuI  PstI  EcoRI XbaI
 MF8                                                       MF10
```

Fig.14

Fig.15

# Fig. 16

European Patent
Office

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | THE JOURNAL OF IMMUNOLOGY, vol. 132, no. 2, February 1984, pages 796-803, The American Association of Immunologists, US; D.H. CONRAD et al.: "The murine lymphocyte receptor for IgE. I. Isolation and characterization of the murine B cell Fc epsilon receptor and comparison with Fc epsilon receptors from rat and human" * Pages 800-803 * | 1-8,40 ,41 | C 12 N 15/00 C 07 K 15/06 C 12 N 1/20 C 12 N 1/18 C 12 P 21/00 C 12 Q 1/68 A 61 K 37/02 |
| X | THE JOURNAL OF IMMUNOLOGY, vol. 129, no. 2, August 1982, pages 563-569, The American Association of Immunologists, US; F.M. MELEWICZ et al.: "Comparison of the Fc receptors for IgE on human lymphocytes and monocytes" * Whole article * | 1-8,40 | |
| X | EUROPEAN JOURNAL OF IMMUNOLOGY, vol. 16, no. 7, July 1986, pages 809-814, VCH Verlagsgesellschaft mbH; T. NAKAJIMA et al.: "IgE receptors on human lymphocytes. I. Identification of the molecules binding to monoclonal anti-Fc epsilon receptor antibodies" * Whole article * | 1-4 | **TECHNICAL FIELDS SEARCHED (Int. Cl 4)**<br>C 12 N<br>C 12 P |

The present search report has been drawn up for all claims

| Place of search | Date of completion of the search | Examiner |
|---|---|---|
| THE HAGUE | 10-04-1987 | CUPIDO M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82